# EUROPEAN PATENT APPLICATION

(11) **EP 4 763 996 A1**
(43) Date of publication of application: **24.06.2026**
(21) Application number: 24854450.4
(22) Date of filing: 13.08.2024
(51) Int. Cl.: C12Q 1/6809, C12Q 1/6827

(54) **METHOD AND KIT FOR SIMULTANEOUSLY DETERMINING COPY NUMBER OF GENE AND GENE VARIANT**

(30) Priority: 16.08.2023 KR 20230106864
(71) Applicant: Genotech Corp., Daejeon 34113 (KR)
(72) Inventor: KIM, Jae Jong, Daejeon 34049 (KR); LIM, Si-Kyu, Gimpo-si, Gyeonggi-do 10068 (KR); KYUNG, Ayoung, Daejeon 34946 (KR); RYU, Jeounghyun, Cheongju-si, Chungcheongbuk-do 28337 (KR); BAEK, Seungwoo, Daejeon 34049 (KR)
(74) Representative: Rimini, Rebecca
(86) International application number: PCT/KR2024/012048
(87) International publication number: WO 2025/037899

(57) **Abstract**

The present invention relates to a real-time quantitative polymerase chain reaction (qPCR) method and kit for simultaneously determining the classification of specific gene variants and the copy number of a gene. More specifically, the present invention relates to an efficient qPCR method and kit for simultaneously determining the variant of a target gene and the copy number of a gene by performing real-time quantitative PCR on a reference gene having a known copy number and the target gene in one reactor in order to determine the copy number of the target gene.

## Description

### Technical Field

The present invention relates to a real-time quantitative polymerase chain reaction (qPCR) method and kit for simultaneously determining discrimination of specific gene variants and a copy number of a gene.

More particularly, the present invention relates to an efficient qPCR method and kit for determining a copy number of a gene by performing real-time quantitative PCR (qPCR) in a single reaction tube with a reference gene having a known copy number and a target gene in order to determine the copy number of the gene, while simultaneously determining a variant type of the target gene.

### Background Art

Extensive human genome studies have shown that genetic variations, including single nucleotide polymorphisms (SNPs) that vary between races and individuals, are closely associated with the pharmacokinetics of drugs. Therefore, information on genetic variations associated with the pharmacokinetics of a patient's prescribed drugs will be very useful for physicians to realize personalized therapy and precision medicine.

Since the 2015 Precision Medicine Initiative in the United States, extensive pharmacogenomic research has been conducted, and the development of individualized diagnostic products utilizing the results is active overseas. NGS (next generation sequencing) and DNA chip-based technologies, which are currently considered the most leading product development technologies, show high false positives and, as equipment-dependent technologies, face difficulties in deriving meaningful clinical trial results and market expansion, necessitating alternative technologies that can overcome these challenges.

Various studies of the human genome have shown that CNVs (copy number variations; hereinafter used interchangeably with "CNV" or "CNVs") are widely distributed, representing approximately 12% of the genome. CNV is a structural variation of the genome that occurs through mechanisms such as insertion, deletion, or duplication of large-sized genes, and CNVs of multiple genes have been found to be associated with genetic diseases, cancer, metabolic diseases, and the like.

Genetic disorders associated with CNV representatively include neurodegenerative disorders such as Parkinson's disease (PD) and early-onset Alzheimer's disease (AD), as well as autoimmune diseases such as systemic lupus erythematosus (SLE), rheumatoid arthritis (RA), Crohn's disease, type 2 diabetes, obesity, susceptibility to HIV infection, and are known to be associated with drug metabolism and toxicity.

Moreover, according *to* recent research results, CNV has been found *to* be involved *in the* onset and progression *of* cancer by causing changes *in* gene expression levels *in* various cancer types, requiring accurate interpretation *of* CNV *in* cancer mechanism and genetic research (Shao et al. BMC Medical Genetics 2019; 20: 175).

The most important genes known to be associated with CNV to date are genes related to the metabolism of drugs and the like. The CNV of these genes is associated with the drug's ADME (absorption, distribution, metabolism, excretion) related to an individual's drug response. The drug's ADME affects pharmacokinetics and pharmacodynamics, thereby causing differences in the drug's efficacy and safety. CNVs of Phase I enzymes related to drug metabolism, such as CYP2A6 (Cytochrome P450 Family 2 Subfamily A Member 6), CYP2D6 (Cytochrome P450 2D6), GSTT1 (glutathione S-transferase theta 1), GSTM1 (Glutathione S-Transferase Mu 1), SULT1A1 (Sulfotransferase Family 1A Member 1), and Phase II enzymes, such as UGT2B 17 (UDP Glucuronosyltransferase Family 2 Member B 17) genes, have been reported to be associated with drug metabolism.

In particular, CYPs such as CYP2D6 and CYP2A6 are distributed in the liver, blood, and the like, and account for approximately 90% of phase I drug metabolism, and are enzymes also involved in the synthesis or metabolism of hormones, cholesterol, vitamin D3, and the like, making them a very important enzyme family in the human body. Numerous SNPs (more than 2000 reported) have been found in human CYPs (57 genes), many of which mutations are associated with drug metabolism, and there are racial differences in the mutation patterns. Drug metabolism can vary not only due to variations in metabolism-related enzymes and the resulting differences in activity, but also according to the number of genes encoding those enzymes. Recent research results report that variations in drug metabolism appear in very complex patterns due to mutations in drug metabolism genes such as CYPs as well as various copy number variations (CNVs).

Depending on the differences in these gene variants (In/Del, SNP, etc.) as well as the differences in CNV number and variation patterns ranging from complete gene deletion, drug metabolism can occur in very complex patterns such as UM (ultra-rapid metabolizer), RM (rapid metabolizer), PM (poor metabolizer), IM (intermediate metabolizer), NM (normal metabolizer), etc. (Wilkinson, G.R.N Engl J Med 2005; 352: 2211-2221).

The CYP2D6 enzyme, one of the most extensively studied CYPs, is known to be involved in the metabolism of 20 to 25% of commercially available drugs (Trends Pharmacol Sci. 2004 Apr; 25(4):193-200. [PubMed: 15063083]), and the activity of the enzyme is known to vary greatly between individuals, being affected by environmental factors and genetic factors. Genetically, CYP2D6 is known to have polymorphisms due to various factors such as SNPs and insertions, deletions, and CNVs resulting therefrom, which become factors causing decrease or increase in drug metabolism.

Moreover, according to recent research results, it is known that *the* transcript of CYP2A7, *a* pseudogene, acts as *a* decoy for microRNA (miR-126) *in the* expression of CYP2A6 (Nakano et al. Drug Metab Dispos. 2015: 43: 703-712), *and the* necessity of accurately measuring *the* copy number of said pseudogene is emerging.

Therefore, diagnosis of the state of CNV, together with determination of genotypes such as SNPs of genes associated with drug metabolism, cancer, and genetic diseases, is very important for more accurately predicting drug metabolism according to the genotype of the target enzyme.

### Detailed Description of the Invention

### Technical Problem

Techniques to accurately measure the CNV of a gene include Next Generation Sequencing (NGS), DNA chip technology, Multiplex Ligation-dependent Probe Amplification (MLPA), and qPCR technology.

The most common method of identifying gene variants and CNVs is NGS technology. This technology determines copy numbers by using read counts in sequence analysis, that is, quantitative analysis information of a sequencing range of a target gene, for an entire genome or multiple target gene groups.

Although this method is suitable for simultaneously analyzing multiple genes, it is not suitable for general laboratory use because expensive equipment is required and substantial cost and time are incurred when analyzing a single assay or a small number of target genes, and it has a disadvantage in that accurate copy number determination is difficult when a gene is homozygous.

Another CNV analysis technique is MLPA (Multiplex Ligation-dependent Probe Amplification) developed by MRC Holland (Schouten et al. Nucleic Acids Res. 2002; 30(12);e57) (U.S. Patent No. 6,955,901 B2).

This technique has not been widely commercialized because it requires expensive equipment, that is, a capillary fragment analyzer, sophisticated sample preparation, and skilled researchers. Moreover, this technique is not well suited for identifying variants.

One of the most useful methods for confirming known gene variants is a qPCR method. This method uses a fluorescent probe specific to a variant sequence, is highly economical, and can be developed in a kit form, thereby making sample preparation and analysis very convenient. In particular, this method is suitable for use in most laboratories performing clinical analysis because analytical equipment, that is, a qPCR system, has become widely available.

Recently, in order to improve specificity and sensitivity of qPCR methods for variant detection, the present inventors have developed a qPCR method that introduces a com-probe competing with a detection probe (Kim, J.J.et al. Sci Rep. 2023; 13: 1036) and a method using a discrimination-boosting (db) oligonucleotide that increases specificity of an allele-specific primer (Korean Registered Patent No. 10-2275038) (Kim, J.J., et al. Sci Rep. 2021; 11: 19098).

One of the most widely known CNV measurement techniques using a qPCR method is a method employing TaqMan probes (Mayo P. et al. Current Protocols in Human Genetics, 2010; 2.13.1-2.13.10). By way of example, a TaqMan Copy Number Assays Kit (Applied Biosystems, Forest City, CA, USA) is commercially available. The kit uses an RNaseP TaqMan Copy Number Reference Assay as an internal control and uses CopyCaller Software (Applied Biosystems, Forest City, CA, USA) to determine copy numbers.

This method is based on relative quantification between a target gene and a reference gene, such as RNaseP, whose exact copy number is known. In this method, qPCR of the target gene and the reference gene is performed separately, a comparative ΔCt of the target gene is obtained, the ΔCt is standardized using a calculator to calculate ΔΔCt, relative quantification (RQ) is determined (RQ= 2^{△△Ct}), and a CNV (= 2 × RQ) of the target gene is determined (Livak K.J. and Schmittgen T.D. Methods 2001; 25: 402-408).

Although this method is a copy number determination technique that retains advantages of the above-described qPCR methods, it requires repeated measurements because copy numbers are measured by statistically processing multiple measurement results obtained from a plurality of PCR reaction vessels, and accuracy is relatively reduced. In addition, this method involves inconvenience in that variant types must be tested through separate reactions, and particularly has a disadvantage in that, when a gene is heterozygous, copy numbers of respective alleles within a diplotype cannot be determined. For example, when a total copy number is three in a heterozygous type, this method cannot distinguish between a Tx1/Gx2 type and a Tx2/Gx1 type.

Since the various copy number determination methods described above require expensive equipment or skilled techniques and also require multiple reactions, there is a need for a method capable of simultaneously determining genotypes together with simple, economical, and highly accurate CNV determination.

### Means for Solving the Problem

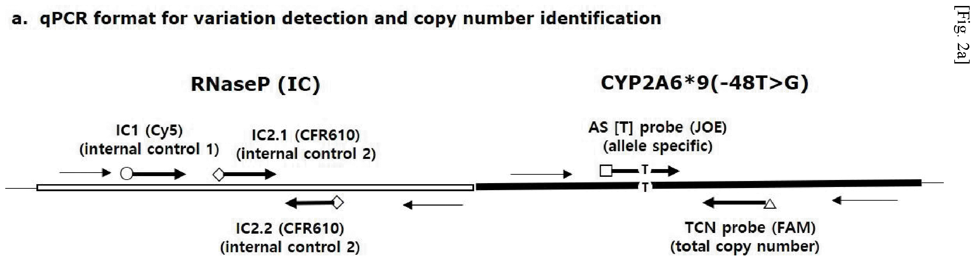

The present disclosure provides a qPCR method for simultaneously determining a copy number of a gene and a genotype of the gene. More specifically, the present disclosure provides a qPCR method capable of identifying gene variations such as single nucleotide polymorphisms (SNPs) and insertions/deletions (In-Dels) within one reaction vessel, and provides a method for comparatively analyzing a copy number of a target gene by simultaneously performing and comparing qPCR with the target gene and a reference gene having a known copy number, for example, RNaseP and genes having no other pseudogenes within a chromosome, including CANX, TBP, YWHAZ, ATP5F1B, and UBC (see Fig. 2a).

For this purpose, the present inventors have developed a method in which, within one reaction vessel, a target gene-specific primer and a target gene-specific probe are used to perform qPCR reflecting a copy number of the target gene while simultaneously performing qPCR amplification of a reference gene using a reference gene-specific primer and probe and comparing amplification results to determine the copy number. In addition, a genotype is simultaneously determined by using an allele-specific probe reflecting a sequence variation (allele type) of the target gene (see Fig. 2a).

Variant sequence typing is determined by confirming presence or absence of signals from an allele-specific probe, and a total copy number of the target gene and/or copy numbers of alleles are calculated by comparing Ct values with a reference gene having a known copy number, for example, RNaseP (see Fig. 2a).

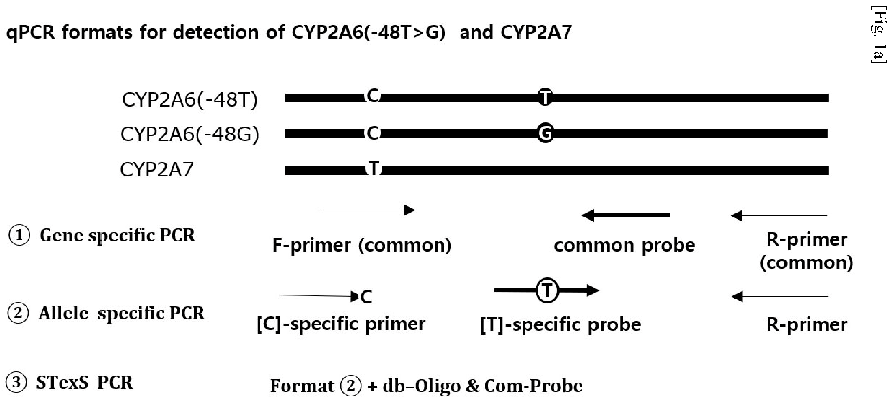

As described above, in the qPCR method according to the present disclosure, PCR using an allele-specific primer or an ARMS primer may be performed to increase specificity of qPCR. In addition, in order to enhance discrimination in detecting minor alleles, a discrimination-enhancing oligonucleotide having an arbitrary sequence configured to partially or fully form a double-stranded structure, as developed by the present inventors (Kim, J.J et al.Sci Rep. 2021; 11: 19098) (hereinafter used interchangeably with "db-Oligo" in the description and drawings), may be added. Furthermore, in order to increase specificity of a probe in the qPCR method according to the present disclosure, a non-specific signal-suppressing Com-probe (hereinafter used interchangeably with "Com-probe" in the description and drawings) may be added, which is complementary to a non-variant target nucleotide sequence, preferentially binds to the non-variant target nucleotide sequence over a variant target nucleotide sequence, and has a structure whose 3' end is not extended by a nucleic acid polymerase (Kim, J.J. et al. Sci Rep. 2023; 13: 1036) (see Fig. 1a).

The oligonucleotide consisting of any sequence that forms *a* double strand partially or entirely, invented by the present inventors for enhancing discrimination in detecting minor alleles, refers to *an* oligonucleotide that comprises *a* forward primer, *a* reverse primer for the target gene, and *an* oligonucleotide that is not complementary to the specific target gene and is capable of reversibly binding to DNA polymerase, wherein the sequence is not specified and consists of any sequence that forms *a* double strand partially or entirely within *a* single strand or with another strand. In the present specification and drawings, it is used interchangeably with "db-Oligo". When this oligonucleotide is used in qPCR, the specificity and sensitivity of qPCR in detecting minor alleles are significantly improved (Kim, J.J., et al. Sci Rep. 2021; 11: 19098).

Furthermore, the non-specific signal-suppressing Com-probe developed by the present inventors will now be described in further detail. A fluorescent hydrolysis probe is designed to match a variant nucleotide sequence of a target gene and differs from a non-variant nucleotide sequence of the target gene by one or more base sequences, preferably one to three base sequences, and more preferably one or two base sequences. In general, when one or more mismatches exist between a probe and a target gene, robustness of hybridization is reduced, such that the probe is displaced before hydrolysis by a 3' nuclease activity of a DNA polymerase occurs during a polymerization process. However, when an excessive amount of a non-variant target gene is present, hydrolysis of a small amount of a non-displaced fluorescent probe may still occur, thereby generating a non-specific signal. In the present disclosure, in order to suppress generation of non-specific signals caused by an excessive amount of the non-variant target gene, a Com-probe having a nucleotide sequence complementary to the non-variant target gene may be added. The Com-probe does not include a fluorescent material and is not linked to a fluorescent material. Since the Com-probe binds to a wild-type (WT) non-variant target gene present in excess in a sample and displacement does not occur, generation of non-specific signals by the fluorescent hydrolysis probe is suppressed. In addition, because the added Com-probe is mismatched with a variant target gene sequence, the Com-probe does not affect hydrolysis of the fluorescent hydrolysis probe matching the variant target gene sequence, thereby significantly facilitating variant detection by qPCR (Kim, J.J. et al. Sci Rep. 2023; 13: 1036).

By qPCR implemented according to the above-described method, accurate determination of a copy number of a target gene and measurement of variant types such as SNPs, enable clear determination of a diplotype of the target gene through determination of copy numbers of respective alleles.

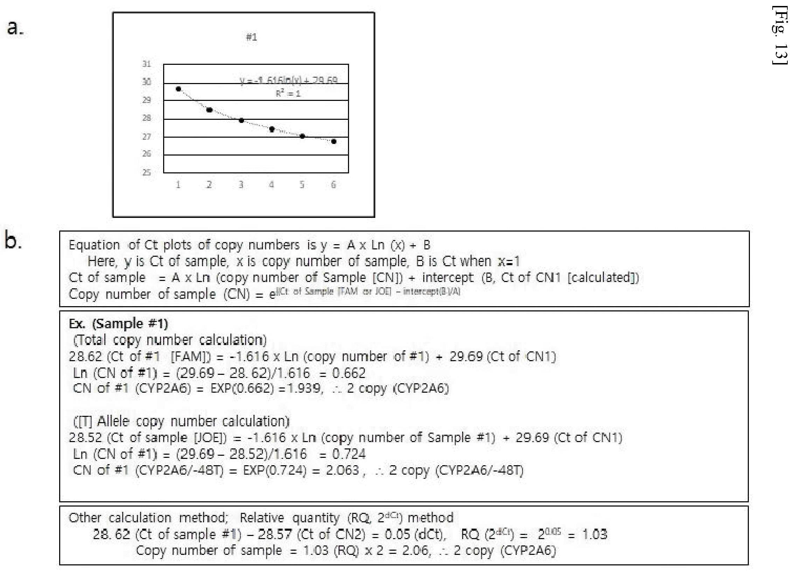

To describe the copy number determination method of the present disclosure in further detail, CN Ct values corresponding to copy numbers 1, 3, 5, and 6 of a target gene are calculated and obtained on the basis of Ct values of copy number 2 (CN2) and copy number 4 (CN4) obtained in each individual experiment. These CN Ct values are substituted to derive a quantitative analysis curve to obtain a function of y = A × ln(x) + B. Then, a Ct value (y) of the target gene is substituted into the function to calculate a copy number (x) of the target gene, thereby determining the copy number as an approximate value (see Fig. 13b).

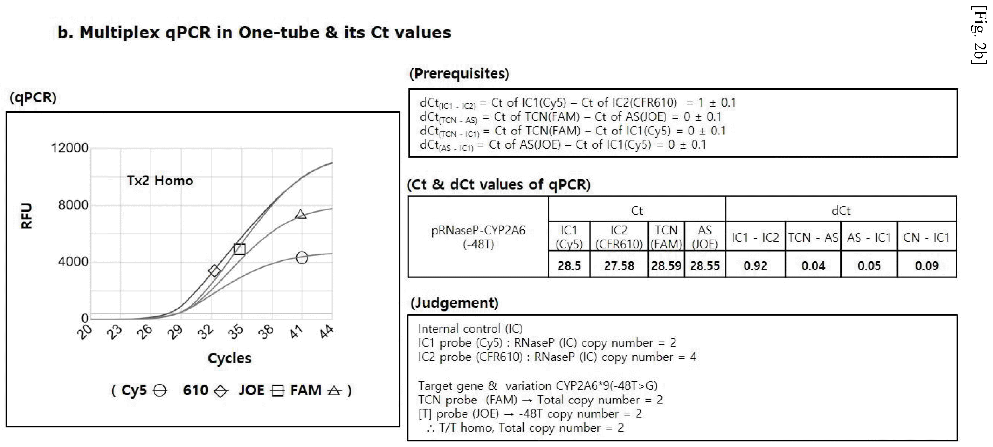

In order to obtain each CN Ct value by the such method, it is preferable to perform qPCR such that Ct value deviations dCt_{(TCN - IC1}) and dCt_{(AS - IC1)} satisfy a condition of 0 ± 0.1 at the same gene copy number in qPCR reactions of a reference gene and a target gene serving as internal controls (see Fig. 2b). Here, dCt_{(TCN - IC1)} refers to a Ct value of TCN minus a Ct value of IC1, and dCt_{(AS - IC1)} refers to a Ct value of AS minus a Ct value of IC1, wherein "IC1" denotes Internal Control 1.

Furthermore, in performing PCR, it is more preferable to perform PCR such that the Ct value of another internal control (IC) probe (CN4; IC2) of the reference gene satisfies the condition that the deviation from the Ct value of CN2, i.e., dCt_{(CN2;IC1-CN4;IC2)}, is 1.0 ± 0.1 so as to reflect that the copy number of the reference gene is twice the copy number of the target gene (see Fig. 2b). Here, dCt_{(CN2;IC1 - CN4;IC2)} refers to the difference between the Ct value of internal control 1 having copy number 2 and the Ct value of internal control 2 having copy number 4. At this time, CN4; IC2 is preferably composed of two probes that emit the same fluorescence and bind to two different regions such that dCt_{(CN2;IC1 - CN4;IC2)} becomes 1.0 ± 0.1, and such two probes are designated as IC2.1 and IC2.2 in the present specification and drawings.

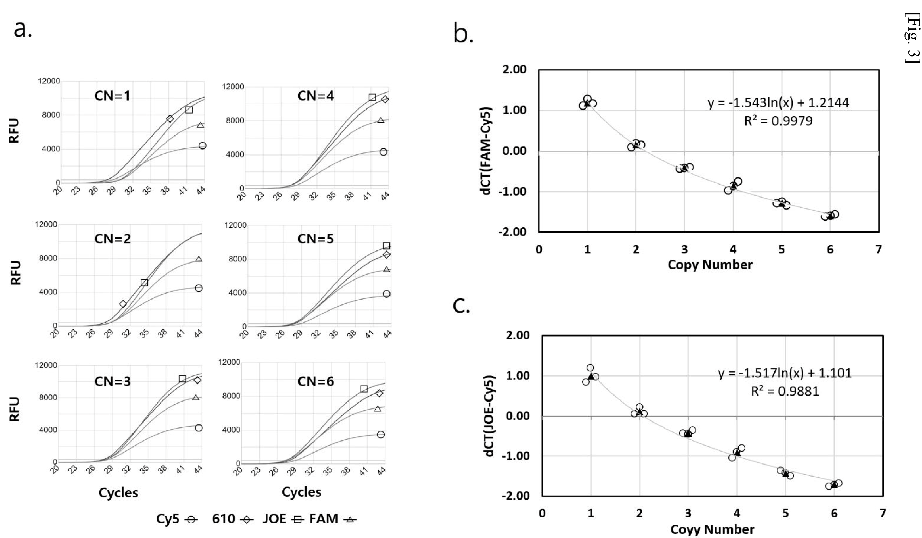

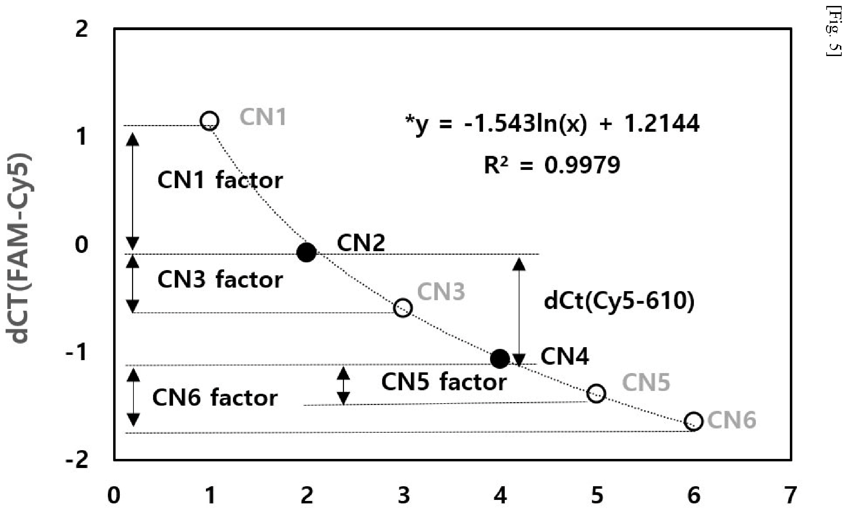

As described above, Ct values obtained from experimental results of samples artificially prepared using standard plasmids for respective copy numbers and subjected to qPCR within one reaction vessel are converted, for synchronizing a target gene and a reference gene, into dCt values defined as (Ct of the target gene - Ct of IC1 of the reference gene), thereby obtaining quantitative curves for respective copy numbers (see Figs. 3b and 3c). Thereafter, a CN Ct factor is determined in order to obtain artificial Ct values corresponding to copy numbers 1, 3, 5, and 6 (see Fig. 5 and Table 5).

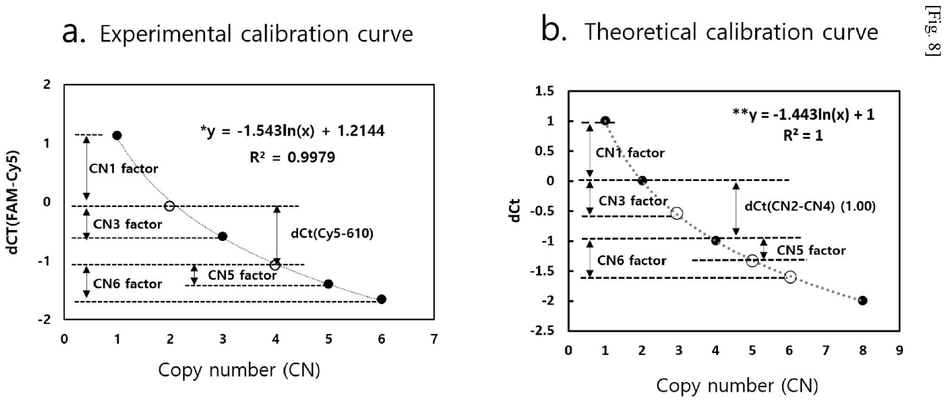

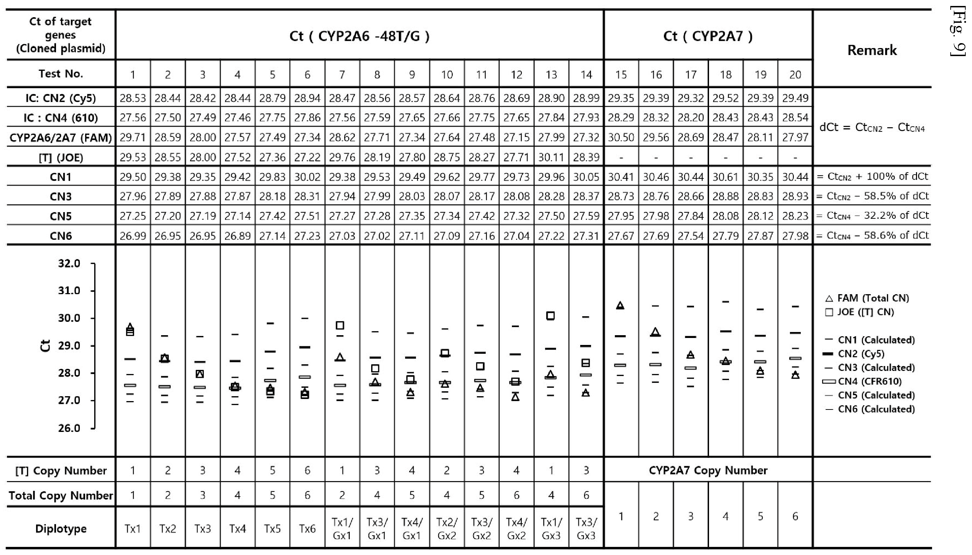

To explain the CN Ct factor assuming theoretically 100% PCR efficiency, using the regression analysis curve equation for Ct value changes according to copy number variation, y = -1.443 × ln(x) + 1 (see Fig. 8b), the CN1 Ct factor [dCt_{(CN1 - CN2)}/dCt_{(CN2 - CN4)}x 100 = 100%], CN3 Ct factor [dCt_{(CN2 - CN3)}/dCt_{(CN2 - CN4)} x 100 = 58.5%], CN5 Ct factor [dCt_{(CN4 - CN5)}/dCt_{(CN2 - CN4}) X 100 = 32.2%], CN6 Ct factor [dCt_{(CN4} - _{CN6)}/dCt_{(CN2 - CN4)} X 100 = 58.6%] are calculated (see Table 7 or Table 10a), and by applying these factors, the CN1 Ct value = Ct_{CN2} + 100% of dCt, CN3 Ct value = Ct_{CN2 -} 58.5% of dCt, CN5 Ct value = Ct_{CN4 -} 32.2% of dCt, CN6 Ct value = CtCN4 ₋ 58.6% of dCt may be obtained (see Fig. 9). Here, dCt represents the difference between the Ct of CN2 and the Ct of CN4 of the reference gene (dCt_{(CN2}-_{CN4)}).

The CN Ct factor and CN Ct values for the experimental values may be obtained in the same way as in the theoretical calculation (see Fig. 5 and Table 5).

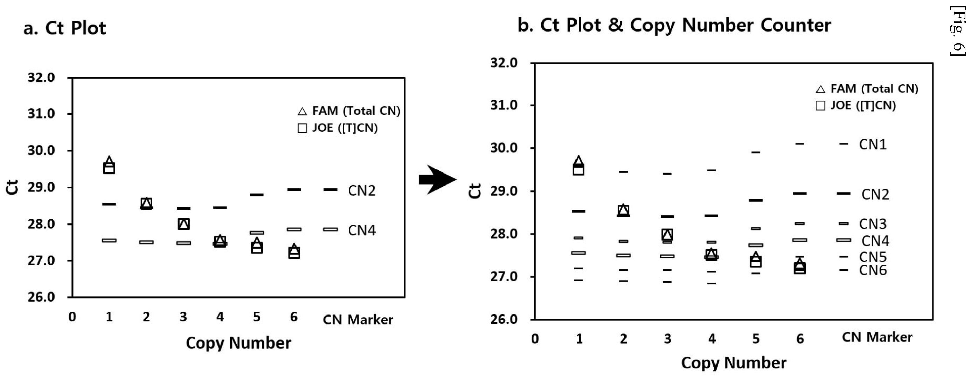

By using the CN Ct factor calculated by the method described above, the Ct values of CN1, CN3, CN5, and CN6 and various information such as CN2, CN4, allele, and total copy number obtained in the experiment may be represented in a single table (see Table 6), and a Ct plot that is visually readable and schematically expressed may be obtained (Fig. 6b).

By applying the CN Ct values obtained by the theoretical method or experimental method described above, a regression analysis curve (standard quantitative curve) equation, y = A × ln(target gene copy number x) + B, may be derived, and the copy number x of the target gene can be obtained by substituting the Ct value y of the analyzed target gene (see Table 10, Table 11).

The method for determining copy number may be calculated and determined from individual tests (see Table 13), or may be calculated and determined from average test values of 3 to 10 repeated tests (see Table 14).

The plot of the present invention can simultaneously display the Ct value of the target gene derived from qPCR performance, the Ct value of the signal corresponding to the allele, the Ct value corresponding to CN2 of the reference gene, and the Ct value corresponding to CN4 of the reference gene (see Fig. 6a), and may display the calculated CN1 Ct value, CN3 Ct value, CN5 Ct value, and CN6 Ct value (see Fig. 6b).

The CN Ct factor presented in the present invention may be used as a constant value without needing to be derived individually for each test, and this enables generation of quantitative curves for CN1, CN2, CN3, CN4, CN5, and CN6 for each individual reaction to accurately determine the copy number of each allele of the target gene diplotype.

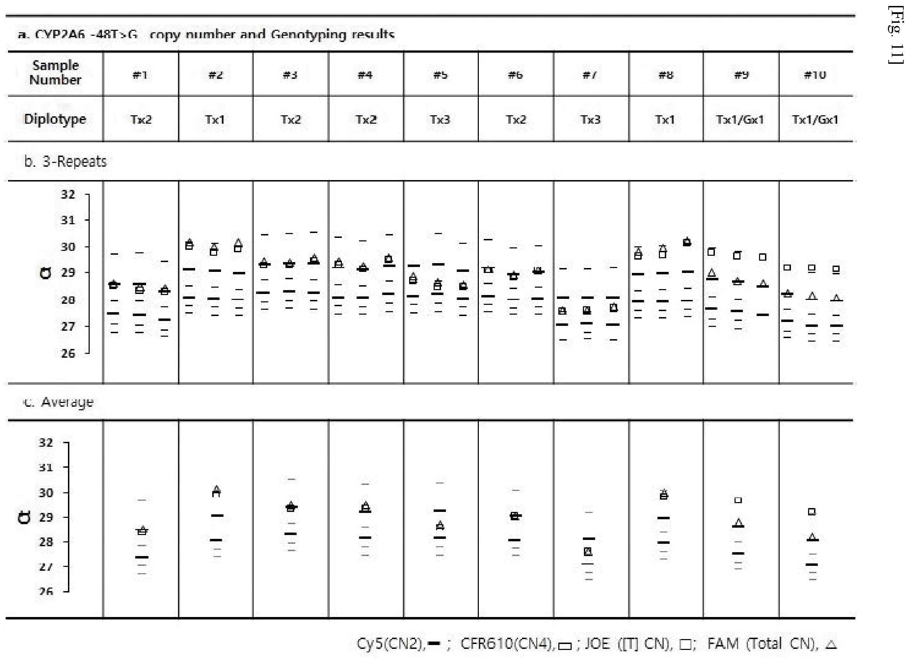

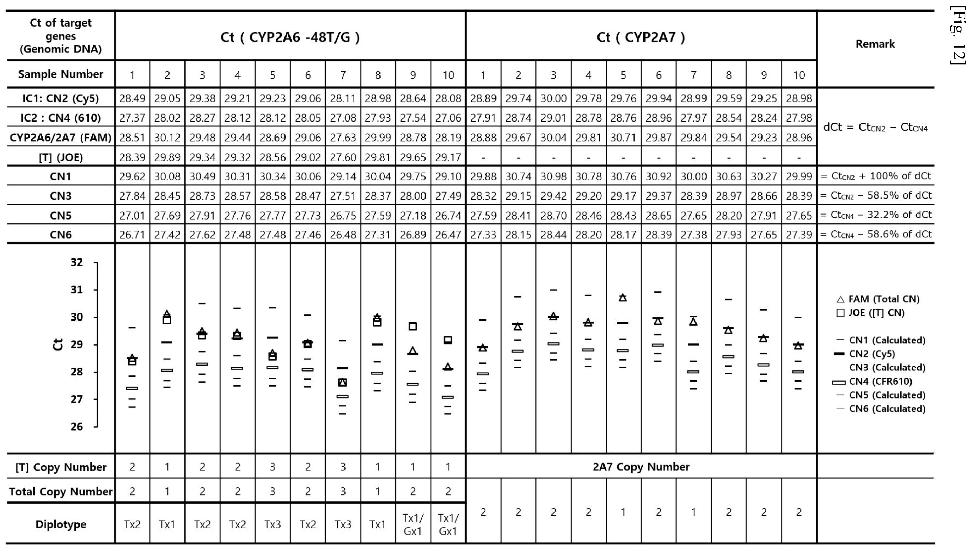

The Ct plot presented in the present invention displays each analyzed sample on one axis, thereby enabling the results of multiple samples to be more clearly distinguished by copy number and diplotype of the samples. Further, the genotype and copy number of the samples may be clearly identified through the test results and calculation of the CNCt value, and if necessary, the pseudogene analysis results may also be visualized to provide simple information, thereby facilitating determination of multiple samples (see FIG. 11 and FIG. 12).

### Effect of invention

The present invention provides a simple qPCR method and kit capable of performing qPCR of a reference gene and a target gene in a single reaction tube and comparing and calculating with the Ct value of the reference gene to simultaneously provide copy number and allelic variant information of the target gene.

### Brief Description of the Drawings

Fig. 1 is a diagram showing establishment of qPCR format and conditions for measurement of CYP2A6 variants and CNV. Fig. 1a shows the configuration and arrangement of primers and probes for three qPCR formats (①, ②, ③) for detection tests of CYP2A6 variants and pseudogene CYP2A7, and Fig. 1b shows the test results (Ct values) for each qPCR format test and the qPCR amplification plots of each qPCR format test.
Fig. 2 is a diagram showing the format of qPCR for variant and CNV determination and the prerequisites for multiplex qPCR performance. Fig. 2a shows the location and configuration of primers and probes for the target gene (CYP2A6), reference gene (RNaseP), and qPCR synchronization, and Fig. 2b shows the prerequisites for qPCR for CNV and variant measurement, test results (qPCR), respective signal values (Ct & dCt values), and judgment using the same.
Fig. 3 is a diagram showing the change in dCt for the reference gene (RNaseP) according to the change in copy number of CYP2A6 (-48T) (homo).
   a. qPCR amplification curves according to changes in the copy number of CYP2A6 (-48T),
   b. regression analysis curve of dCt_{(FAM - Cy5)} according to changes in the copy number of CYP2A6 (-48T),
   c. regression curve of dCt_{(JOE - CY5)} according to changes in the copy number of CYP2A6 (-48T).
Fig. 4 is a qPCR amplification curve according to changes in the copy number of CYP2A6 (-48T/G) (heterozygous).
Fig. 5 is a diagram illustrating the calibration curve and CN factors for copy number and dCt values.
Fig. 6 shows the Ct curve according to copy number variation and a Ct plot applying calculated Ct values for each copy number. a is a vertical Ct graph of qPCR signals according to copy number, and b is a Ct graph applying calculated CN Ct values for use as a copy number counter.
Fig. 7 shows the change in dCt for the reference gene (RNaseP) according to the change in CYP2A7 copy number. a is the qPCR amplification curve according to the change in CYP2A7 copy number, and b 6B is the graph of the dCt value (FAM - Cy5) according to the change in CYP2A6 (-48T) copy number.
Fig. 8 shows a comparison of the CN factors calculated from experimental values and the CN factors calculated from theoretical values. a shows the graph of dCt_{(FAM-Cy5)} and CN factors according to the copy number obtained by experiment, and b shows the graph of dCt_{(FAM-Cy5)} and CN factors according to the change in the theoretical copy number.
Fig. 9 is a diagram showing the results of SNP and CNV tests using standard plasmids, and the determination of Ct graphs and diplotypes. The copy number was measured using the values calculated from Table 9, and the diplotypes were determined therefrom. Test Nos. 1 to 5; tests and results of Example 5 (Table 3), Test Nos. 6 to 14: tests and results of Example 6 Table 4, Test Nos. 15 to 20; tests and results of Example 10 (Table 9) were used.
Fig. 10 is a qPCR amplification curve for measuring variants and copy number of CYP2A6 and pseudogene CYP2A7 of human genomic DNA. 2 ng of genomic DNA was used and other qPCR conditions were the same as in Example 13, and only one representative example of each of the 3 replicate tests is presented.
Fig. 11 is a diagram showing the results of SNP and CNV determination using human genomic DNA and the Ct graph and diplotype determination using the same. a is a table of copy number and diplotype determination, b is the results of the 3 replicate qPCR tests for each sample, and c shows the test average values thereof.
Fig. 12 is a diagram showing the results of SNP and CNV determination using human genomic DNA and the determination of Ct graphs and diplotypes. The results of the 3 replicate qPCR tests of the samples are shown and Fig. 12C shows the test average values thereof.
Fig. 13 shows an equation for calculating the copy number and an example of the calculation (human genome DNA sample 1). a is a graph equation derived using the Ct value of sample 1 in Fig. 8, and b is a calculation method using the derived graph equation.

### Best Mode for Carrying Out the Invention

An aspect of the present invention relates to a method for simultaneously determining a copy number and a genotype of a gene, comprising:
a) obtaining amplification experimental values (Ct values) by qPCR by simultaneously adding to one reaction tube: i) a target gene-specific primer, a target gene-specific probe, and a probe capable of distinguishing alleles of the target gene; and ii) a reference gene-specific primer and probe capable of amplifying and detecting a reference gene, and synchronizing amplification of the genes;
b) determining a copy number Ct factor (hereinafter also referred to as a "CN Ct factor") from the amplification experimental values and/or theoretical amplification values;
c) determining Ct values of target gene copy numbers 1, 3, 5 and/or 6 from amplification experimental values of copy number 2 and copy number 4 of the reference gene by using the determined copy number Ct factor; and
d) determining a total copy number of the target gene and a genotype of alleles by comparing amplification experimental values of the target gene with the Ct values of copy numbers 1, 3, 5 and/or 6 of the target gene determined in step c) and the test copy number of the reference gene.

An aspect of the present invention also relates to the method for simultaneously determining a copy number and a genotype of a gene, wherein, in step a), in i), a primer and a probe capable of specifically amplifying a pseudogene of the target gene are further added.

An aspect of the present invention also relates to the method for simultaneously determining a copy number and a genotype of a gene, wherein at least one of the target gene-specific primer and the pseudogene-specific primer is an AS primer (Allele specific primer) or an ARMS primer (Amplification Refractory Mutation System primer).

An aspect of the present invention also relates to the method for simultaneously determining a copy number and a genotype of a gene, wherein, in the qPCR step of step a), a discrimination-enhancing oligonucleotide that is not complementary to the primers and the template, is capable of reversibly binding to a DNA polymerase for qPCR, and is configured to form a partially or fully double-stranded structure within a single strand or with another strand, is additionally introduced to enhance discrimination of specific primer amplification; and a non-specific signal-suppressing probe that is complementary to a target nucleotide sequence of a non-variant target gene, preferentially binds to the target nucleotide sequence of the non-variant target gene over a target nucleotide sequence of a variant target gene, and has a structure whose 3' end is not extended by a nucleic acid polymerase, is additionally introduced to enhance discrimination of a specific probe.

An aspect of the present invention also relates to the method for simultaneously determining a copy number and a genotype of a gene, wherein, in the qPCR step of step a), for confirming a copy number of the target gene and an allele copy number, qPCR is performed using a target gene-specific probe and an allele-specific probe that are fluorescent probes having different wavelengths.

An aspect of the present invention also relates to the method for simultaneously determining a copy number and a genotype of a gene, wherein, in the qPCR step of step a), for obtaining amplification experimental values of copy number 2 and copy number 4 of the reference gene, qPCR is performed using fluorescent probes having different wavelengths.

An aspect of the present invention also relates to the method for simultaneously determining a copy number and a genotype of a gene, wherein, in step b), the determining of the copy number Ct factor from the amplification experimental values is performed by deriving a calibration curve using experimental Ct values of a standard gene and determining copy number Ct factors for copy numbers 1, 3, 5 and/or 6 of the target gene.

An aspect of the present invention also relates to the method for simultaneously determining a copy number and a genotype of a gene, wherein, in step b), the determining of the copy number Ct factor from the theoretical amplification values is performed using Equation y = -1.443 × ln(x) + 1, wherein y is a Ct value of the target gene and x is a copy number of the target gene.

An aspect of the present invention also relates to the method for simultaneously determining a copy number and a genotype of a gene, wherein, in step c), the determining of Ct values of target gene copy numbers using the copy number Ct factor is performed by calculating a difference (dCt CN2-CN4) between a Ct of copy number 2 and a Ct of copy number 4 of the reference gene obtained from amplification experimental values of the target gene and the reference gene having known copy numbers, and applying the copy number Ct factor obtained in claim 6 or 7 to determine Ct for copy numbers 1, 3, 5 and/or 6 of the target gene.

An aspect of the present invention also relates to the method for simultaneously determining a copy number and a genotype of a gene, wherein the comparing of amplification experimental values of the target gene with Ct values of target gene copy numbers is performed by deriving a standard quantitative curve equation, y = A × ln(x) + B, by applying Ct values of copy numbers 1, 3, 5 and/or 6 of the target gene and a CN2 Ct value and a CN4 Ct value of the target gene, wherein y is a Ct value of the target gene and x is a copy number of the target gene, and determining copy numbers of the target gene and alleles using the equation.

An aspect of the present invention also relates to the method for simultaneously determining a copy number and a genotype of a gene, wherein the synchronizing amplification of the target gene and the reference gene is performed by conducting qPCR with the target gene and the reference gene located on a single plasmid.

An aspect of the present invention also relates to the method for simultaneously determining a copy number and a genotype of a gene, wherein the performing qPCR by synchronizing amplification of the target gene and the reference gene is carried out by adjusting qPCR conditions so that Ct value deviations dCt(TCN - IC1) and dCt(AS - IC1) each satisfy ±0.1 at the same gene copy number for both genes in the qPCR reaction of the reference gene and the target gene.

An aspect of the present invention also relates to the method for simultaneously determining a copy number and a genotype of a gene, wherein qPCR is performed such that the copy number of the reference gene is twice the copy number of the target gene.

An aspect of the present invention also relates to the method for simultaneously determining a copy number and a genotype of a gene, wherein qPCR is performed by adjusting qPCR conditions so that dCt(CN2;IC1 - CN4;IC2) satisfies 1.0 ± 0.1 when the copy number of the reference gene is twice the copy number of the target gene.

An aspect of the present invention also relates to the method for simultaneously determining a copy number and a genotype of a gene, wherein, in the qPCR step, for obtaining amplification experimental values of copy number 4, two probes having different sequences and the same fluorescence are simultaneously used.

An aspect of the present invention also relates to the method for simultaneously determining a copy number and a genotype of a gene, wherein, for obtaining amplification experimental values of copy number 4 of the target gene, a Ct value that is 1.0 less than a Ct of copy number 2 of the target gene is arbitrarily substituted and calculated as a Ct of copy number 4. The general copy number is 2, and the Ct of copy number 4, which is twice the copy number, can be calculated as the Ct value of copy number 4 by arbitrarily substituting a Ct value that is 1.0 less than the Ct value of copy number 2.

An aspect of the present invention also relates to a method for preparing a simultaneous determination table of a gene copy number and a genotype, comprising displaying qPCR Ct results and copy number Ct values on one axis, displaying target gene samples to be analyzed on another axis, and displaying the table such that a genotype and a copy number of one or more analysis samples are visually identifiable.

An aspect of the present invention also relates to the method for preparing a simultaneous determination table of a gene copy number and a genotype, wherein the determination table comprises Ct values, Ct values by copy number, and copy numbers of diplotypes and allele types.

An aspect of the present invention also relates to the method for preparing a simultaneous determination table of a gene copy number and a genotype, wherein the determination table further comprises a copy number of a pseudogene.

An aspect of the present invention also relates to the method for preparing a simultaneous determination table of a gene copy number and a genotype, wherein the determination table reflects result values and/or calculated values obtained from the method for simultaneously determining a copy number and a genotype of a gene.

An aspect of the present invention also relates to a simultaneous determination table of a gene copy number and a genotype, displaying qPCR Ct results and copy number Ct values on one axis and displaying target gene samples to be analyzed on another axis such that a genotype and a copy number of one or more analysis samples are visually identifiable.

An aspect of the present invention also relates to the simultaneous determination table of a gene copy number and a genotype, wherein the determination table comprises Ct values, Ct values by copy number, and copy numbers of diplotypes and allele types.

An aspect of the present invention also relates to the simultaneous determination table of a gene copy number and a genotype, wherein the determination table further comprises a copy number of a pseudogene.

An aspect of the present invention also relates to a qPCR kit for simultaneous determination of a copy number and a genotype of a target gene, comprising:
(a) a target gene-specific primer and a target gene-specific probe;
(b) a specific probe capable of discriminating and detecting alleles of the target gene;
(c) a reference gene-specific primer and a reference gene-specific probe capable of amplifying and verifying a reference gene; and
(d) a DNA polymerase.

An aspect of the present invention also relates to a qPCR kit for simultaneous determination of a copy number and a genotype of a target gene, comprising:
(a) a target gene-specific primer and a target gene-specific probe;
(b) a specific probe capable of discriminating and detecting alleles of the target gene;
(c) a reference gene-specific primer and a reference gene-specific probe capable of amplifying and verifying a reference gene;
(d) a primer and a probe capable of specifically amplifying a pseudogene of the target gene; and
(e) a DNA polymerase.

An aspect of the present invention also relates to qPCR kit, further comprising:
(f) a discrimination-enhancing oligonucleotide that is not complementary to the primers and a template of the target gene, is capable of reversibly binding to a DNA polymerase for qPCR, and is configured to form a partially or fully double-stranded structure within a single strand or with another strand; and
(g) a non-specific signal-suppressing probe that is complementary to a target nucleotide sequence of a non-variant target gene, preferentially binds to the target nucleotide sequence of the non-variant target gene over a target nucleotide sequence of a variant target gene, and has a structure whose 3' end is not extended by a nucleic acid polymerase.

### Mode for Carrying Out the Invention

Hereinafter, the configuration of the present invention will be described in more detail with reference to specific Examples and Experimental Examples. However, it will be apparent to those skilled in the art to which the present invention pertains that the scope of the present invention is not limited to the descriptions of the Examples and Experimental Examples. Unless otherwise specified, the terms used in the present invention are intended to have meanings commonly used by those skilled in the art to which the present invention pertains.

In addition, in the Examples of the present invention, FAM, JOE, CFR610, and Cy5 were used as fluorescent materials for identification; however, the fluorescent materials are not limited only to the materials exemplified above, and it will be apparent to those skilled in the art that each sequence-labeled fluorescent material may be substituted with different fluorescent materials.

### Example 1: Construction of Standard Plasmids for Testing

As a reference gene for CNV measurement, the housekeeping gene RNaseP was obtained and incorporated into a plasmid vector as constructed below. Using human genomic DNA (Promega) as a template, the RNaseP gene was obtained by PCR amplification using Pfu DNA polymerase (Enzynomics, Korea) with a forward primer RNaseP-F (5'-GGTACCGCTGGCACTTTCTGTTATG-3', SEQ ID NO: 1) and a reverse primer RNaseP-R (5'-ACTAGTGGGAGAAGAAGGAAGGGA-3', SEQ ID NO: 2). The amplified product was inserted into a pTOP Blunt V2 vector and transformed into *E. coli* DH5α to obtain a plasmid into which the RNaseP gene had been inserted. The RNaseP sequence (SEQ ID NO: 26) of the obtained plasmid was confirmed by nucleotide sequencing, and a confirmed pRNaseP plasmid was constructed. The constructed pRNaseP plasmid was digested with a restriction enzyme *Not*I and used in experiments. A vector was constructed such that CYP-related genes for CNV measurement were positioned downstream of the pTOP-RNaseP vector. Using human genomic DNA (Promega) of CYP2A6, CYP2A7, and CYP2D6 as templates, vectors were obtained by PCR amplification using Pfu DNA polymerase (Enzynomics, Korea). For CYP2A6, primers CYP2A6-F (5'-CCACAGCCCTGCGGCACC-3', SEQ ID NO: 3) and CYP2A6-R (5'-TGGTCAACCCCCTGCCAC-3', SEQ ID NO: 4) were used; for CYP2A7, primers CYP2A7-F (5'-TCACCCTAATAAAACCCAGACCT-3', SEQ ID NO: 5) and CYP2A7-R (5'-GGACCCGGATGCTGAAACT-3', SEQ ID NO: 6) were used; and for CYP2D6, primers CYP2D6-F (5'-ACAGTCAACACAGCAGGTTCA-3', SEQ ID NO: 7) and CYP2D6-R (5'-CTGTTTCATGTCCACGACCC-3', SEQ ID NO: 8) were used. Amplification products of each gene were inserted into a pTOP Blunt V2 vector to construct plasmids. The sequences of the constructed CYP2A6 (SEQ ID NO: 27 or SEQ ID NO: 28) and CYP2A7 (SEQ ID NO: 29) were confirmed, and an allele variant plasmid pCYP2A6(-48T) was constructed. The finally obtained plasmids were pCYP2A6(-48T), pCYP2A6(-48G), and pCYP2A7. In addition, in order to match copy numbers of a reference gene and target genes to be analyzed in these plasmids, a reference gene RNaseP was linked as a single unit as described below and used. The pRNaseP vector was digested with *Kpnl*/*Spel* to obtain a fragment (456 bp), and the fragment was cloned into each gene plasmid digested with *Kpnl*/*Spel* to obtain pRNaseP/CYP2A6(-48T), pRNaseP/CYP2A6(-48G), and pRNaseP/CYP2A7. The obtained plasmids were digested with restriction enzymes to produce single-stranded forms, purified, and used as standard plasmids for CNV and variant measurement.

### Example 2: qPCR Performance

qPCR (real-time quantitative PCR) was performed using a CFX Opus 96 Real-Time PCR System for 45 cycles after an initial incubation at 95°C for 10 minutes, followed by cycling at 95°C for 20 seconds and 60°C for 40 seconds. The primers and probes used for qPCR for each target were of the types and amounts presented in each Example. The reaction mixture comprised 3 U of Taq polymerase (STexS-TaqII, GenoTech, Korea) and a buffer (5× STexS-DB1.2 Taq buffer or 5× GenoTaq buffer, GenoTech, Korea), adjusted to a total volume of 20 µL. The Ct values of the qPCR were determined at a threshold value of 400. Plasmids were used at a quantified concentration of 10⁴ copies/µL, and human genomic DNA was used at 1 ng/µL. The human genomic DNA was obtained from the Daejeon Biomedical Regulatory-Free Zone Human-Derived Material Bank (IRB file No. CNUH 2022-08-047).

### Example 3: Determination of qPCR Format

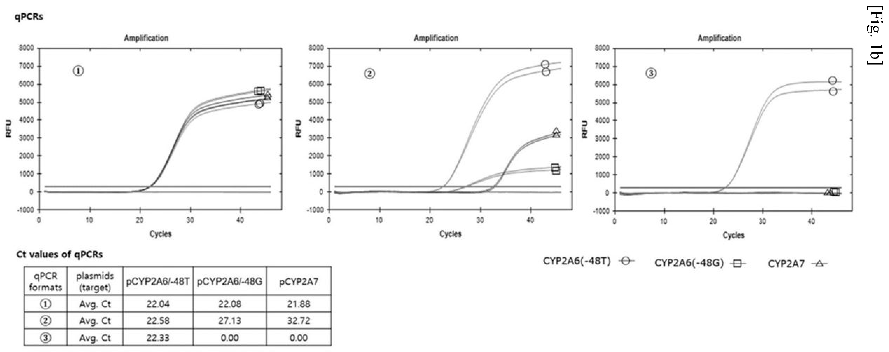

As an example for testing the present invention, copy number measurement and allele typing were performed for CYP2A6*9 (-48T>G). In the human genome, a pseudogene CYP2A7 (SEQ ID NO: 29) that is highly similar to CYP2A6 (SEQ ID NO: 27) is present. Therefore, an appropriate qPCR format capable of clearly discriminating between the two genes is required. To this end, three formats were compared, as shown in Table 1 and FIG. 1a: a general qPCR using primers, probes, and buffer (format ①); a method employing a specific primer for suppressing amplification of the pseudogene CYP2A7 (format ②); and a highly discriminatory qPCR format employing the STexS PCR method (see Kim, J.J. et al. Sci Rep. 2023; 13: 1036), using STexS-DB1.2 Taq buffer (GenoTech, Korea), for suppressing non-specific signals (format ③). Using pCYP2A6(-48T), pCYP2A6(-48G), and pCYP2A7 plasmids as templates, qPCR was performed according to each of the formats, and the results are shown in FIG. 1b. The qPCR results of format ① exhibited nearly identical Ct values for all genes, thereby providing no discrimination. In contrast, when a specific primer whose 3' terminal sequence confers discrimination between CYP2A6 and CYP2A7 (CYP2A6-S-F, SEQ ID NO: 12) was used together with a -48T-specific probe (FAM-CYP2A6-48T, SEQ ID NO: 13), the respective genes were distinguished to a significant extent.

However, significant levels of qPCR signals were also observed in CYP2A7 and CYP2A6(-48G). For complete suppression of these non-specific signals, the applicant's STexS technology was applied. When 5x STexS-DB1.2 Taq buffer (Genotech, Korea) was applied to suppress amplification of CYP2A7 occurring despite the use of the specific primer (CYP2A6-S-F, SEQ ID NO: 12) and a corresponding com-probe (CYP2A6/-48G-Com-P SEQ ID NO: 14) was used to suppress non-specific binding of the specific probe (FAM-CYP2A6-48T SEQ ID NO: 13), the non-specific signals of the CYP2A7 (SEQ ID NO: 29) and CYP2A6-48G (SEQ ID NO: 28) genes observed when the STexS technology was applied were completely suppressed, and there was no change in *the* qPCR Ct value derived from CYP2A6-48T (SEQ ID NO: 27).

Table 1 below shows the sequences, Tm values and dosages of primers, probes, etc. for the qPCR format test.

**[Table 1]**

| qPCR format | primer/probe/etc. | sequence (5' → 3') (Tm) | pmoles /20ul |
|---|---|---|---|
| Format ① | CYP2A6/2A7-F1 (SEQ ID NO: 9) (F-primer) | 5'-AGGTIATTATGTAATIAGCCAAAGTCC (63.7°C) | 10 |
| | AS-RI (SEQ ID NO: 10) (R-primer) | 5'-ACAGTCAGGCAGICCAGCAAGG (65.82°C) | 10 |
| | FAM-R-CYP2A6/CNV5.2 (SEQ ID NO:11) (common probe) | 5'-FAM- CTGAGGCCAGCATGGTGGTAGTG-BHQ1 (68.3°C) | 10 |
| format ② | CYP2A6-S-F (SEQ ID NO: 12) ([C]-specific primer) | 5'- GGAGGTGAAATGAGGTAATTATGTAGTC (64.4°C) | 10 |
| | AS-RI (SEQ ID NO: 10) (R-primer) | 5'-ACAGTCAGGCAGICCAGCAAGG (65.82°C) | 10 |
| | FAM-CYP2A6-48T (SEQ ID NO: 13) ([T]-specific probe) | 5'-FAM-TTCAGGCAGTATAAAGGCAAAC- BHQI (58.4°C) | 10 |
| Format ③ | CYP2A6-S-F (SEQ ID NO: 12) (F-primer) | 5'- GGAGGTGAAATGAGGTAATTATGTAGTC (64.4°C) | 10 |
| | AS-R1 (SEQ ID NO: 10) (R-primer) | 5'-ACAGTCAGGCAGICCAGCAAGG (65.82°C) | 10 |
| | FAM-CYP2A6-48T (SEQ ID NO: 13) ([T]-specific probe) | 5'-FAM-TTCAGGCAGTATAAAGGCAAAC-BHQ1 (58.4°C) | 10 |
| | CYP2A6/-48G-Com-P (SEQ ID NO: 14) (Com-Probe) | 5'-TTCAGGCAGTAGAAAGGCAAACp (60.1°C) | 20 |

| | | | |
|---|---|---|---|
| *I is inosine. | | | |

### Example 4: Performance of multiplex qPCR for copy number determination and variant discrimination

Using the qPCR format③ established in Example 3, a qPCR system for measuring the genotype and copy number of CYP2A6*9 (-48T>G) was constructed. For this purpose, the representative housekeeping gene RNaseP was first selected as a reference gene. Since this gene exists as one copy per strand in the human chromosome (i.e., 2N, thus actually two copies), to implement this, the pRNaseP/CYP2A6(-48T) standard plasmid in which RNaseP and CYP2A6 genes were linked as shown in FIG. 2a was used as the single plasmid of Example 1.

This was used as a template to perform qPCR with the primer/probe set and conditions as shown in Table 2. For PCR harmonization of the reference gene RNaseP and the target gene CYP2A6(-48T), the amount, position, and length of each primer and probe were adjusted to determine the Ct value of each gene. At this time, the IC1 (internal control 1) probe for detecting the reference gene RNaseP was configured to correspond to a Ct value of CN = 2, and the IC2 (internal control 2, IC2.1 + IC2.2) probe was configured to correspond to a Ct value of CN = 4, which is twice the copy number.

The prerequisites for PCR harmonization are to establish qPCR conditions satisfying dCt_{(ICI-IC2)}= Ct of ICl(Cy5) - Ct of IC2(CFR610) = 1 ± 0.1 and dCt_{(TCN-As)}= Ct of TCN(FAM) - Ct of AS(JOE) = 0 ± 0.1. When qPCR satisfying these prerequisites is performed to obtain Ct values and dCt values for each probe (Ct & dCt values of qPCR), judgment can be made as described below. The Ct value of the IC1 probe (Cy5: CN2) indicates that the copy number (CN) of RNaseP is 2, and the Ct value of the IC2 probe (CFR610: CN4) (IC2.1 + IC2.2) indicates that the copy number of RNaseP is 4. The Ct value of the TCN-probe-FAM corresponding to the target gene (CYP2A6) reflects that the copy number of CYP2A6 is 2 because it is linked on one plasmid, and in the case of the [T] probe (CYP2A6-48T-JOE), since it reflects a T base sequence at the -48 position, it reflects that the copy number of CYP2A6(-48T) is 2.

This provides a very important indication, i.e., that it is possible to easily measure the copy number within one reaction tube when performing qPCR against a reference gene of a known copy number (e.g., RNaseP in this embodiment) and a target gene of an unknown copy number (e.g., CYPs) that are physically connected.

Table 2 shows primer/probe set information for the CYP2A6(-48T) copy number assay.

**[Table 2]**

| CYP2A6 | | Sequence (5' → 3') (Tm) | pmoles /20ul |
|---|---|---|---|
| CYP2A6/-48T/G | CYP2A6-S-F (SEQ ID NO: 12) | 5'- GGAGGTGAAATGAGGTAATTATGTAGTC (64.4°C) | 10 |
| | AS-R1 (SEQ ID NO: 10) | 5'-ACAGTCAGGCAGICCAGCAAGG (65.82°C) | 10 |
| | CYP2A6-48T-JOE (SEQ ID NO: 15) | 5'-JOE-TTCAGGCAGTATAAAGGCAAAC- BHQ1 (58.4°C) | 20 |
| | TCN-probe-FAM (SEQ ID NO: 16) | 5'-FAM- CCTGAGGCCAGCATGGTGGTAGTG-BHQ1 (70.6°C) | 10 |
| | CYP2A6/-48G-Com-P | 5'-TTCAGGCAGTAGAAAGGCAAACp (60.1 °C) | 9 |
| RNaseP | RNaseP_F6 (SEQ ID NO: 17) | 5'-AGGCTTGCTGTTTGGGCTCT (60.5 °C) | 10 |
| | RNaseP-R2 (SEQ ID NO: 18) | 5'-TGACTTGCTTTTAACATGACCATTC (60.9 °C) | 10 |
| | IC1 (Cy5) (SEQ ID NO: 19) | 5'-Cy5-AGTGACGCCAAGGCTGCGGTG- BHQ3 (67.3°C) | 6 |
| | IC2.1 (CFR610) (SEQ ID NO: 20) | 5'-CFR610-TGCCGAGCAGCGCTTCT-BHQ2 (57.3 °C) | 9 |
| | IC2.2 (CFR610) (SEQ ID NO: 21) | 5'-CFR610- TGTACTGGAAGAACTTGCTTAC-BHQ2 (58.4 °C) | 9 |

### Example 5: Correlation between dCt changes of the reference gene (RNaseP) and the target gene (CYP2A6) and copy number

The changes in Ct values of each probe were confirmed by proportionally increasing the amount of the target gene CYP2A6 compared to the reference gene RNaseP. For this purpose, qPCR was performed as in Example 2 using the independently prepared standard plasmids pRNaseP and pCYP2A6(-48T) with the primer and probe set of Table 2 (Table 3 and Fig. 3). As a result of the test, the dCt(Ct_{(IC1)(Cy5)} - Ct_{(IC2)(CFR610)}) of IC1 and IC2 showed stable measurement values with an average of 0.99, similar to dCt = 1.0 when the theoretical copy number was 2-fold, and Ct_{(TCN)(FAM)} - Ct_{(IC1)(Cy5)} and Ct_{(TCN)(JOE)} - Ct_{(IC1)(Cy5)} clearly decreased with the proportional increase of pCYP2A6 reflecting the copy number. In particular, dCt_{(FAM - Cy5)} and dCt_{(JOE - Cy5)} according to copy number showed similar calibration curves (y = -1.543 x ln(x) + 1.2144 [R² = 0.9881] and y = -1.517 x ln(x) + 1.101 [R² = 0.9979]) (Fig. 3b and c).

Table 3 shows changes in dCt for the reference gene (RNaseP) according to changes in CYP2A6(-48T) copy number.

**[Table 3]**

| Copy Number (CN) | Plasmid* | | Ct** | | | | dCt | | | |
|---|---|---|---|---|---|---|---|---|---|---|
| | pRN-aseP | pCY P2A 6(-48T) | IC1 (Cy5: CN2) | IC2 (CFR 610: CN4) | TCN (CYP 2A6) (FAM) | AS([T]) (JOE) | IC1 - IC2 (Cy5 - C FR610) | | TCN - IC1 (FAM-Cy5) | AS - IC1 (JOE-Cy5) |
| 1 | 2ul | 1 ul | 28.53 | 27.56 | 29.71 | 29.53 | 0.97 | Average 0.991 | 1.18 | 1.00 |
| 2 | | 2ul | 28.44 | 27.50 | 28.59 | 28.55 | 0.94 | | 0.14 | 0.11 |
| 3 | | 3ul | 28.42 | 27.49 | 28.00 | 28.00 | 0.93 | | -0.42 | -0.42 |
| 4 | | 4ul | 28.44 | 27.46 | 27.57 | 27.52 | 0.98 | | -0.87 | -0.92 |
| 5 | | 5ul | 28.79 | 27.75 | 27.49 | 27.36 | 1.04 | | -1.30 | -1.43 |
| 6 | | 6 ul | 28.94 | 27.86 | 27.34 | 27.22 | 1.08 | | -1.60 | -1.72 |

| | | | | | | | | | | |
|---|---|---|---|---|---|---|---|---|---|---|
| *Each plasmid was tested at 1 x 10⁵ copies/µl. **Ct values are averages of 3 replicates measured at Threshold 400. | | | | | | | | | | |

The test results showing the amplification curve and regression analysis curve are presented in FIG. 3.

### Example 6: Effect of Ct Value Change According to Copy Number Variation on Allelic Contamination

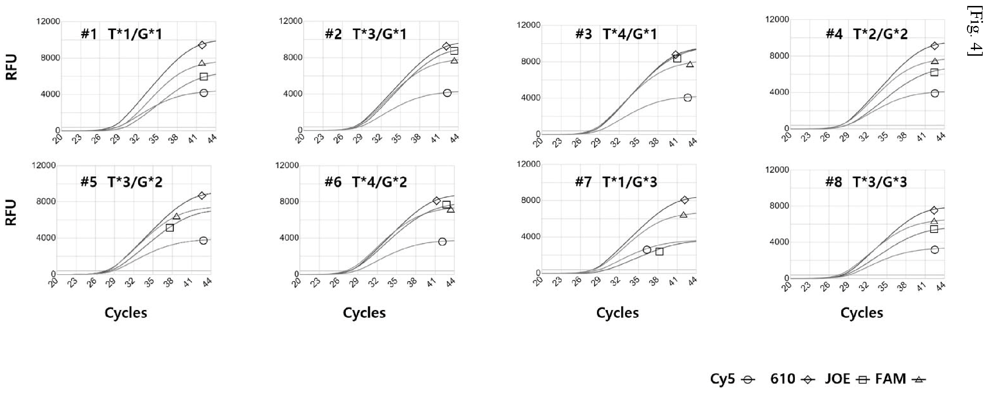

pRNaseP, pCYP2A6(-48T) and pCYP2A6(-48G) were used as standard plasmids (each 1 x 10⁵ copies/µl). Each plasmid was mixed and tested assuming various diplotypes and SNP types. As a result, the dCt_{(Cy5-CFR610)} values averaged 0.993 (STDEV = 0.059), satisfying the predetermined condition of 1 ± 0.1 as in Example 5, and showed stable increases and decreases in Ct reflecting the copy number (Table 4 and Fig. 4). These results demonstrate that genetic variant typing and copy number measurement can be performed simultaneously even when alleles are mixed.

Table 4 shows the Ct values of qPCR according to changes in CYP2A6(-48T/G) (heterozygote) copy number.

**[Table 4]**

| No | Diplotypes | C N | Plasmids* | | | Ct** | | | | dCt | |
|---|---|---|---|---|---|---|---|---|---|---|---|
| | | | pRN-aseP | pCY P2A6 (-4 8T) | pCY P2A6 (-48G) | IC1 (Cy5: CN2) | IC2 (C FR610:CN4) | TCN (CYP 2A6) (FAM) | AS([T]) (JOE) | IC1 - IC2 (CY5 - C FR610) | |
| #1 | T*1/ G*1 | 2 | 2ul | 1 ul | 1 ul | 28.47 | 27.56 | 28.62 | 29.76 | 0.91 | |
| #2 | T*3/ G*1 | 4 | 2ul | 3ul | 1 ul | 28.56 | 27.59 | 27.71 | 28.19 | 0.97 | |
| #3 | T*4/ G*1 | 5 | 2ul | 4ul | 1 ul | 28.57 | 27.65 | 27.34 | 27.80 | 0.92 | |
| #4 | T*2/ G*2 | 4 | 2ul | 2ul | 2ul | 28.64 | 27.66 | 27.64 | 28.75 | 0.98 | Average 0.993 |
| #5 | T*3/ G*2 | 5 | 2ul | 3ul | 2ul | 28.76 | 27.75 | 27.48 | 28.27 | 1.01 | |
| #6 | T*4/ G*2 | 6 | 2ul | 4ul | 2ul | 28.69 | 27.65 | 27.15 | 27.71 | 1.04 | |
| #7 | T*1/ G*3 | 4 | 2ul | 1 ul | 3ul | 28.90 | 27.84 | 27.99 | 30.11 | 1.06 | |
| #8 | T*3/ G*3 | 6 | 2ul | 3ul | 3ul | 28.99 | 27.93 | 27.32 | 28.39 | 1.06 | |

| | | | | | | | | | | | |
|---|---|---|---|---|---|---|---|---|---|---|---|
| *Each plasmid was tested using 1 x 10⁵ copies/µl. ** The Ct values are averages of 3 replicates measured at Threshold 400. | | | | | | | | | | | |

### Example 7: Calculation of Ct Factor and Ct Value Using Calibration Curves

In order to calculate more sophisticated Ct values according to copy number in conjunction with the Ct of a reference gene (RNaseP) having a known copy number, a correlation calibration curve between the copy number of the target gene and the dCt_{(FAM-}C_{y5)} of the target gene and reference gene was prepared using the results of the test of Example 5 (FIG. 5). The calculated dCt(calculated) of each copy number calculated using the equation y = -1.543 x ln(x)+ 1.2144 derived from this calibration curve is shown in Table 5. Using this, the ratio of the difference in Ct values with the reference gene having a known copy number was determined as CN1 factor, CN3 factor, CN5 factor, and CN6 factor as shown in the Ct factors of Table 5. These factors were calculated as percentages of the average value (0.991) of dCt_{(JOE-}C_{FR610)} of IC1 and IC2 obtained in Example 5 to obtain values linked to the difference between the Ct values of CN2 (Cy5) or CN4 (CFR610) of the reference gene (RNaseP) and the target gene (CYP2A6) (Table 5). From the obtained Ct factors, Ct values for each copy number (CN Ct values) may be calculated by adding or subtracting from the Ct values of CN2 (Cy5) or CN4 (CFR610) of RNaseP as shown in the method for obtaining Ct values in Table 5.

**[Table 5]**

| CN | | 1 | 2 | 3 | 4 | 5 | 6 |
|---|---|---|---|---|---|---|---|
| *dCt_{(FAM-Cy5)} | | 1.18 | 0.14 | -0.42 | -0.87 | -1.30 | -1.60 |
| **dCt_{(calculated)} | | 1.214 | 0.145 | -0.481 | -0.925 | -1.269 | -1.550 |
| ***CN Ct factor | CN1 Ct factor | (1.214 - 0.145)/0.991 x 100 = 107.87 (%) | | | | | |
| | CN3 Ct factor | (0.145 + 0.481)/0.991 x 100 = 63.17 (%) | | | | | |
| | CN5 Ct factor | (-0.925 + 1.269)/0.991 x 100 = 34.71 (%) | | | | | |
| | CN6 Ct factor | (-0.925 + 1.550)/0.991 x 100 = 63.07 (%) | | | | | |
| ****CN Ct value | CN1 Ct Value | CN2 Ct_{(Cy5)} + dCt_{(Cy5 - CFR610)} x 107.87 % (CN1 Ct factor) | | | | | |
| | CN3 Ct Value | CN2 Ct_{(Cy5)} - dCt_{(Cy5 - CFR610)} x 63.17 % (CN3 Ct factor) | | | | | |
| | CN5 Ct Value | CN4 Ct_{(CFR610)} - dCt_{(cy5} - _{CFR610)} x 34.71 % (CN5 Ct factor) | | | | | |
| | CN6 Ct Value | CN4 Ct_{(CFR610)} - dCt_{(cy5} - _{CFR610)} x 63.07% (CN6 Ct Factor) | | | | | |

| | | | | | | | |
|---|---|---|---|---|---|---|---|
| *dCt is the result obtained from the test of Example 5. **The calculated dCt is a value obtained by calculation using the equation y = - 1.543 x ln(X) +1.2144 of FIG. 5. ***The CN Ct factor was expressed as a percentage of the average value (0.991) of dCt _{(JOE-CFR610)}, which is the difference between the Ct value of IC1 (CY5) and the Ct value of IC2 (CFR610) of the reference gene obtained from the test, relative to the calculated dCt value. ****CN Ct value is a value calculated by adding or subtracting the Ct value corresponding to each copy number to or from the Ct value of the corresponding copy number of the reference gene (RNaseP). | | | | | | | |

### Example 8. Calculation of Ct factor and Ct Value Using a Calibration Curve

Each Ct factor was obtained using the test values of Example 5 (Table 3) by the method presented in Example 7, and the Ct values of CN1, CN3, CN5, and CN6 were calculated by applying the factors (Table 6). These values may be displayed on one axis together with the Ct test values for each test group (copy number) and represented as shown in FIG. 6b. In such a Ct plot, by additionally displaying the calculated Ct values corresponding to each CN in FIG. 6a where only the test values are displayed, the test values corresponding to each copy number may be readily compared with the CNCt values corresponding to each copy number, thereby providing highly useful results for copy number determination.

**[Table 6]**

| Diplotype | CN | Ct* | | | | Ct value (calculated) ** | | | |
|---|---|---|---|---|---|---|---|---|---|
| | | IC:CN 2(Cy5) | IC:C N4 (CF R610) | CYP2A6 (FAM) | T(JOE) | Ct factors | | | |
| | | | | | | 107.87 % | 63.17% | 34.71% | 63.07% |
| | | | | | | CN1 | CN3 | CN5 | CN6 |
| T*1 | 1 | 28.53 | 27.56 | 29.71 | 29.53 | 29.58 | 27.92 | 27.22 | 26.95 |
| T*2 | 2 | 28.44 | 27.50 | 28.59 | 28.55 | 29.45 | 27.85 | 27.17 | 26.91 |
| T*3 | 3 | 28.42 | 27.49 | 28.00 | 28.00 | 29.42 | 27.83 | 27.17 | 26.90 |
| T*4 | 4 | 28.44 | 27.46 | 27.57 | 27.52 | 29.50 | 27.82 | 27.12 | 26.84 |
| T*5 | 5 | 28.79 | 27.75 | 27.49 | 27.36 | 29.91 | 28.13 | 27.39 | 27.09 |
| T*6 | 6 | 28.94 | 27.86 | 27.34 | 27.22 | 30.10 | 28.26 | 27.49 | 27.18 |

| | | | | | | | | | |
|---|---|---|---|---|---|---|---|---|---|
| *This is the test value of Example 5. **Ct values calculated according to the method presented in Table 5. | | | | | | | | | |

### Example 9. Comparison between Experimental Values and Theoretical Values of Calibration Curves

The calibration curves (experimental values) presented in Examples 5 and 7 and the derived equation y = -1.543 x ln(x) + 1.2144 and each CN Ct factor (experimental values) calculated therefrom were compared and examined with theoretical values, i.e., a theoretical calibration curve (theoretical values) and CN Ct factors (theoretical values) calculated using CN2 assuming 100% PCR amplification efficiency and dCt values of respective copy numbers to confirm the usefulness thereof.

The theoretical calibration curve is as shown in Fig. 8B, and the function of the logarithmic graph at that time is y = -1.443 xln(x) + 1. Based on the experimental values, the calculated values and theoretical values were similar: CN1 Ct factor was 107.87% and 100%, CN3 Ct factor was 63.17% and 58.5%, CN5 Ct factor was 34.71% and 32.2%, and CN6 Ct factor was 63.07% and 58.6%, respectively (Tables 5 and 7).

Table 7 below shows the equation for the change of dCt according to the change in the theoretical copy number and the Ct factor calculated using the equations.

**[Table 7]**

| CN | 1 | 2 | 3 | 4 | 5 | | 6 | 8 |
|---|---|---|---|---|---|---|---|---|
| Theoretical dCt | 1 | 0 | - | -1 | - | | - | -2 |
| Calculated dCt* | - | - | -0.585 | - | -1.322 | | -1.586 | - |
| CN1 Ct factor | dCt_{(CN1} - _{CN2)}/dCt_{(CN2} - _{CN4)} X 100 | | | | | 100 (%) | | |
| CN3 Ct factor | dCt_{(CN2} - _{CN3)}/dCt_{(CN2 - CN4)} X 100 | | | | | 58.5 (%) | | |
| CN5 Ct factor | dCt_{(CN4} - _{CN5)}/dCt_{(CN2 - CN4)} X 100 | | | | | 32.2 (%) | | |
| CN6 Ct factor | dCt_{(CN4} - _{CN6)}/dCt_{(CN2 - CN4)} X 100 | | | | | 58.6 (%) | | |

| | | | | | | | | |
|---|---|---|---|---|---|---|---|---|
| *These values are calculated using the equation y = -1.443 × ln(CN) + 1, and the equation is obtained as a logarithmic function plot of the dCt correlation between each copy number CN and CN2, assuming 100% PCR amplification efficiency. | | | | | | | | |

### Example 10. Correlation between dCt Changes of the Reference Gene (RNaseP) and CYP2A7 and Copy Number

Accurate calculation of the copy number of CYP2A7, which is a pseudogene of CYP2A6, is required. For this purpose, simultaneous evaluation of the copy number of CYP2A7 is necessary. In order to measure the accurate copy number of only CYP2A7 while excluding CYP2A6, testing was performed using the primer and probe set of Table 8, which includes an ARMS primer (CYP2A7-F1-2, SEQ ID NO:) capable of amplifying only CYP2A7.As a result of the qPCR, amplification of the pCYP2A6 gene did not occur, and qPCR was performed using pRNaseP/CYP2A7 under qPCR conditions satisfying the prerequisite dCt_{(FAM-Cy5)} = 0 ± 0.1 presented in Example 4.

**[Table 8]**

| **primer/probes** | | **Sequence (5'→ 3') (Tm)** | **pmole/20ul** |
|---|---|---|---|
| CYP2A7 | CYP2A7-F1-2 | 5'- TGTAGTTGGGAGGTGAAATAAGTTG62.1 °C) | 20 |
| | AS primer 1 | 5'-ACAGTCAGGCAGICCAGCAAGG (65.82°C) | 20 |
| | FAM-R-CYP2A6/CN V5.1 | 5’-FAM-CCTGAGGCCAGCATGGTGGTAG TG-BHQ1 (70.6°C) | 6 |
| RNaseP | RnaseP_F6 | 5' -AGGCTTGCTGTTTGGGCTCT (60.5 °C) | 10 |
| | RnaseP-R2 | 5'-TGACTTGCTTTTAACATGACCATTC (60.9 °C) | 10 |
| | Cy5-Rnase P-1.2.2 | 5'-Cy5-TGACGCCAAGGCTGCGG-BHQ3 (59.8 °C) | 10 |
| | g610-R-CNV2 | 5'-CFR610- GTGTACTGGAAGAACTTGCTTACCT- BHQ2 (62 °C) | 10 |

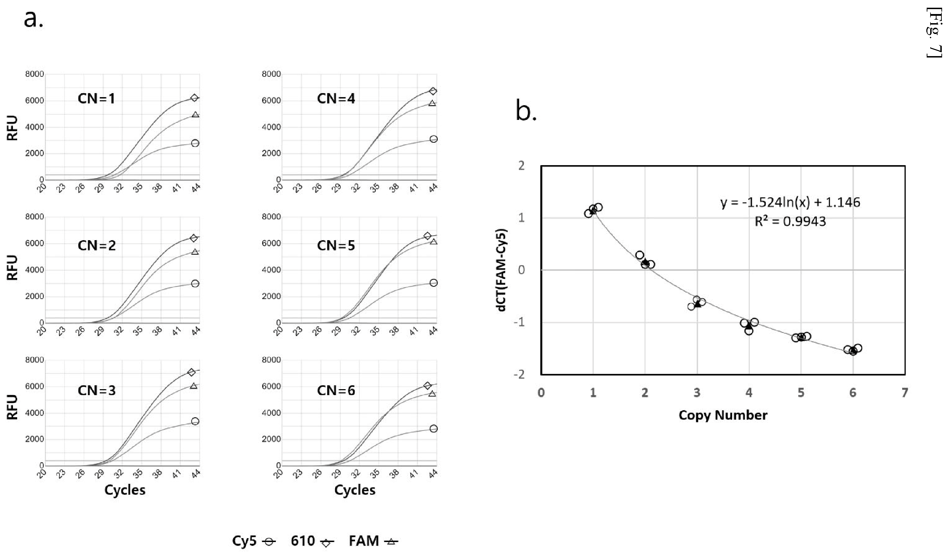

The Ct test values of each probe were obtained by adding the pCYP2A7 plasmid as a template to achieve copy numbers of 1 to 6 relative to the reference gene RNaseP (Table 9). As a result of this test, the dCt_{[Ct(IC1)Cy5-Ct(IC2)(CFR610)]} of IC1 and IC2 showed an average of 1.04, which represents stable measurement values similar to dCt=1.0 when the theoretical copy number was 2-fold, and Ct_{(TCN)(FAM)} - Ct_{(IC1)(Cy5)} clearly decreased according to the proportional increase of pCYP2A6 reflecting the copy number. In particular, the dCt _{(FAM-Cy5)} calibration curve according to copy number (y = -1.524 X ln (x) + 1.146 [R² = 0.9943] (Fig. 7B)) showed similarity to the test result of CYP2A6 in Example 5, y = -1.543 x ln (x) + 1.2144 (Fig. 5).

Table 9 shows changes in dCt for the reference gene (RNaseP) according to changes in CYP2A7 copy number.

**[Table 9]**

| CN | Plasmid* | | Ct* * | | | dCt | | |
|---|---|---|---|---|---|---|---|---|
| | pRNaseP | pCY P2A7 | IC: CN2 (Cy5) | IC: CN4 (CFR 610) | CYP 2A7 (FAM) | Cy5 - CFR610 | | FAM - Cy5 |
| 1 | | 1 ul | 29.35 | 28.29 | 30.50 | 1.06 | | 1.15 |
| 2 | | 2ul | 29.39 | 28.32 | 29.56 | 1.06 | | 0.18 |
| 3 | 2ul | 3ul | 29.32 | 28.20 | 28.69 | 1.11 | Average 1.04 | -0.63 |
| 4 | | 4ul | 29.52 | 28.43 | 28.47 | 1.09 | | -1.05 |
| 5 | | 5ul | 29.39 | 28.43 | 28.11 | 0.96 | | -1.28 |
| 6 | | 6 ul | 29.49 | 28.54 | 27.97 | 0.95 | | -1.52 |

| | | | | | | | | |
|---|---|---|---|---|---|---|---|---|
| *Each plasmid was tested using 1 x 10⁵ copies/ul. **Ct values are 3-replicate averages measured at Threshold 400. | | | | | | | | |

### Example 11: Comparison of Copy Number Calculation Using Experimental and Theoretical CN Ct Factors

Copy numbers were calculated using the experimental CN Ct factors and the theoretical CN Ct factor, and the calculated copy numbers were compared to examine the usefulness thereof. The experimental CN Ct factors were obtained using the tests of Example 5 and Example 10. From the test of Example 5 using CYP2A6, a CYP2A6 CN Ct factor (FAM) (②) was obtained using FAM signal values, and a CYP2A6 [T] allele CN Ct factor (JOE) (③) was obtained using JOE signal values. In addition, from the test of Example 10 using pCYP2A7, a CYP2A7 CN Ct factor (FAM) (④) (Table 10) was obtained using the same method. The CN Ct values were calculated by applying the respective CN Ct factors of ②, ③, and ④ thus obtained and the theoretical CN Ct factor (①), and the results are shown in Table 11.

Using the Ct values of each CN calculated in this way, a logarithmic function (y = A x ln (x) + B) was derived and used to accurately calculate the Ct values. In this equation, y is the Ct value of the sample, A is the factor of the natural logarithm term, and the constant B represents the copy number, i.e., the Ct value when x = 1. Based on this, the copy number of the target gene can be calculated using the qPCR Ct value of the target gene, i.e., the Ct value of the FAM signal value (total copy number, TCN) or the JOE signal value ([T] allele copy number), and based on this, the copy number can be determined (Table 11). The calculated values of such copy numbers and the determination results based thereon showed that the determination results using the theoretical Ct factor and using the Ct factor derived from experimental values were identical in all tests, and the determined copy numbers were identical to the test conditions and results (Table 11).

Table 10 shows the CN Ct factors, and Table 11 compares the copy number calculations by applying the CN Ct factors.

**[Table 10]**

| | **① Theoretica l CN Ct factor** | **②Experimenta l Total CYP2A6 CN Ct factor (FAM)** | **③ Experimental CYP2A6 [T] Allele CN Ct factor (JOE)** | **④ Experimental Total CYP2A7 CN Ct factor (FAM)** |
|---|---|---|---|---|
| **CN1 Ct factor** | 100% | 107.91 % | 105.60 % | 100.61 % |
| **CN3 Ct factor** | 58.5 % | 63.17 % | 61.77 % | 58.85 % |
| **CN5 Ct factor** | 32.2% | 34.74 % | 34.00 % | 32.39 % |
| **CN6 Ct factor** | 58.6 % | 63.12% | 61.77 % | 58.85 % |

**[Table 11]**

| **Experimental Ct & Calculated CN Ct values** | | | | | | | | | | **Copy Number Calculation** | | | | | | |
|---|---|---|---|---|---|---|---|---|---|---|---|---|---|---|---|---|
| **CYP2A6(-48T>G)*** | | | | | | | | | | **y= A x ln(x) + B** | | **Total Copy Number** | | **[T] Allele Copy Number** | | |
| **Ct fac tor** | No. | FAM | JOE | CN1 | CN2 (Cy5) | CN3 | CN4 (610) | CN5 | CN6 | A | B | Calcul ated | **Judge ment** | Calcu lated | **Judge ment** | |
| ① | 1 | 29.71 | 29.53 | 29.50 | 28.53 | 27.96 | 27.56 | 27.25 | 26.99 | -1.400 | 29.500 | 0.861 | **1** | 0.979 | **1** | |
| | 2 | 28.59 | 28.55 | 29.38 | 28.44 | 27.89 | 27.50 | 27.20 | 26.95 | -1.356 | 29.380 | 1.791 | **2** | 1.844 | **2** | |
| | 3 | 28.00 | 28.00 | 29.35 | 28.42 | 27.88 | 27.49 | 27.19 | 26.95 | -1.342 | 29.350 | 2.735 | **3** | 2.735 | **3** | |
| ② | 1 | 29.71 | - | 29.58 | 28.53 | 27.92 | 27.56 | 27.22 | 26.95 | -1.458 | 29.558 | 0.901 | **1** | - | - | |
| | 2 | 28.59 | - | 29.45 | 28.44 | 27.85 | 27.50 | 27.17 | 26.91 | -1.413 | 29.436 | 1.820 | **2** | - | - | |
| | 3 | | 28.00 | - | 29.42 | 28.42 | 27.83 | 27.49 | 27.17 | 26.90 | -1.398 | 29.405 | 2.733 | **3** | - | - |
| ③ | 1 | | - | 29.53 | 29.55 | 28.53 | 27.93 | 27.56 | 27.23 | 26.96 | -1.441 | 29.541 | - | **-** | 1.008 | **1** |
| | 2 | | - | 28.55 | 29.43 | 28.44 | 27.86 | 27.50 | 27.18 | 26.92 | -1.397 | 29.420 | - | **-** | 1.864 | **2** |
| | 3 | | - | 28.00 | 29.40 | 28.42 | 27.85 | 27.49 | 27.17 | 26.92 | -1.382 | 29.389 | - | **-** | 2.733 | **3** |

| **CYP2A7**** | | | | | | | | | | | **y = A x ln(x) +** B | | **Total Copy Number** | | - | |
|---|---|---|---|---|---|---|---|---|---|---|---|---|---|---|---|---|
| **Ct fac tor** | No. | FAM | | JOE | CN 1 | CN 2 (Cy5) | CN 3 | CN 4 (610) | CN 5 | CN 6 | A | B | ted | Calcula**Judge ment** | | |
| ① | 1 | 30.50 | | - | 30.41 | 29.35 | 28.73 | 28.29 | 27.95 | 27.67 | -1.530 | 30.410 | 0.943 | **1** | | |
| | 2 | 29.56 | | - | 30.46 | 29.39 | 28.76 | 28.32 | 27.98 | 27.69 | -1.544 | 30.460 | 1.791 | **2** | | |
| | 3 | 28.69 | | - | 30.44 | 29.32 | 28.66 | 28.20 | 27.84 | 27.54 | -1.616 | 30.440 | 2.953 | **3** | | |
| ④ | 1 | 30.50 | | - | 30.42 | 29.35 | 28.73 | 28.29 | 27.95 | 27.67 | -1.534 | 30.415 | 0.946 | **1** | | |
| | 2 | 29.56 | | - | 30.47 | 29.39 | 28.76 | 28.32 | 27.97 | 27.69 | -1.549 | 30.465 | 1.794 | **2** | | |
| | 3 | 28.69 | | - | 30.45 | 29.32 | 28.66 | 28.20 | 27.84 | 27.54 | -1.621 | 30.445 | 2.953 | **3** | | |

| | | | | | | | | | | | | | | | | |
|---|---|---|---|---|---|---|---|---|---|---|---|---|---|---|---|---|
| *The test results of Nos. 1, 2, and 3 of Example 5 (Table 3) were used. **A value tested using pCYP2A7. | | | | | | | | | | | | | | | | |

### Example 12: Method for Presenting Results for SNP and CNV Determination and Usefulness Thereof

The present inventors sought to present a determination system for determining CNV and genotype (SNP) in a more efficient and visually useful manner. FIG. 9 is an exemplary drawing for this purpose. As shown in FIG. 9, the present invention is a system configured to obtain each Ct value obtained through qPCR testing and CN Ct values calculated from the Ct values, and to diagram the same so as to clearly present the same according to each test group. In the drawing, determination result values were calculated according to the method of Example 11 (Table 12). The result analysis of FIG. 9 and Table 12 used tests and results of Example 5 (Table 3), tests and results of Example 6 (Table 4), and tests and results of Example 10 (Table 9). Such a method of result analysis, presentation of analysis results, and determination has an advantage in clearly presenting information on pseudogenes, information on copy numbers, and genotype (SNP) information for a plurality of test samples.

Table 12 is a copy number calculation table showing calibration curves derived for copy number calculation and copy numbers calculated therefrom.

**[Table 12]**

| **CYP2A6 -48T>G** | | | | | | | | | **y = A x ln (x) +B** | | **Total Copy Number** | | **[T] Allele Copy Number** | |
|---|---|---|---|---|---|---|---|---|---|---|---|---|---|---|
| Test No. | **FAM** | **JOE** | CN 1 | **CN 2 (Cy5)** | CN 3 | **CN 4 (610)** | CN 5 | CN 6 | A | B | Calcul ated | **Jud gem ent** | Calcu lated | **Jud gem ent** |
| 1 | 29.71 | 29.53 | 29.50 | 28.53 | 27.96 | 27.56 | 27.25 | 26.99 | -1.400 | 29.500 | 0.861 | **1** | 0.979 | **1** |
| 2 | 28.59 | 28.55 | 29.38 | 28.44 | 27.89 | 27.50 | 27.20 | 26.95 | -1.356 | 29.380 | 1.791 | **2** | 1.844 | **2** |
| 3 | 28.00 | 28.00 | 29.35 | 28.42 | 27.88 | 27.49 | 27.19 | 26.95 | -1.342 | 29.350 | 2.735 | **3** | 2.735 | **3** |
| 4 | 27.57 | 27.52 | 29.42 | 28.44 | 27.87 | 27.46 | 27.14 | 26.89 | -1.414 | 29.420 | 3.700 | **4** | 3.833 | **4** |
| 5 | 27.49 | 27.36 | 29.83 | 28.79 | 28.18 | 27.75 | 27.42 | 27.14 | -1.501 | 29.830 | 4.756 | **5** | 5.186 | **5** |
| 6 | 27.34 | 27.22 | 30.02 | 28.94 | 28.31 | 27.86 | 27.51 | 27.23 | -1.558 | 30.020 | 5.583 | **6** | 6.030 | **6** |
| 7 | 28.62 | 29.76 | 29.38 | 28.47 | 27.94 | 27.56 | 27.27 | 27.03 | -1.313 | 29.380 | 1.784 | **2** | 0.749 | **1** |
| 8 | 27.71 | 28.19 | 29.53 | 28.56 | 27.99 | 27.59 | 27.28 | 27.02 | -1.400 | 29.530 | 3.671 | **4** | 2.605 | **3** |
| 9 | 27.34 | 27.80 | 29.49 | 28.57 | 28.03 | 27.65 | 27.35 | 27.11 | -1.328 | 29.490 | 5.051 | **5** | 3.572 | **4** |
| 10 | 27.64 | 28.75 | 29.62 | 28.64 | 28.07 | 27.66 | 27.34 | 27.09 | -1.414 | 29.620 | 4.056 | **4** | 1.850 | **2** |
| 11 | 27.48 | 28.27 | 29.77 | 28.76 | 28.17 | 27.75 | 27.42 | 27.16 | -1.457 | 29.770 | 4.813 | **5** | 2.799 | **3** |
| 12 | 27.15 | 27.71 | 29.73 | 28.69 | 28.08 | 27.65 | 27.32 | 27.04 | -1.501 | 29.730 | 5.580 | **6** | 3.842 | **4** |
| 13 | 27.99 | 30.11 | 29.96 | 28.90 | 28.28 | 27.84 | 27.50 | 27.22 | -1.530 | 29.960 | 3.626 | **4** | 0.907 | **1** |
| 14 | 27.32 | 28.39 | 30.05 | 28.99 | 28.37 | 27.93 | 27.59 | 27.31 | -1.530 | 30.050 | 5.959 | **6** | 2.961 | **3** |

| CYP2A7 | | | | | | | | | ***y* = A x ln(*x*) +B** | | **Total Copy Number** | | | |
|---|---|---|---|---|---|---|---|---|---|---|---|---|---|---|
| Test No. | **FAM** | **JOE** | CN 1 | **CN 2 (Cy5)** | CN 3 | **CN 4 (610)** | CN 5 | CN 6 | A | B | Calcul ated | **Jud gem ent** | | |
| 15 | 30.50 | - | 30.41 | 29.35 | 28.73 | 28.29 | 27.95 | 27.67 | -1.530 | 30.410 | 0.943 | **1** | - | |
| 16 | 29.56 | - | 30.46 | 29.39 | 28.76 | 28.32 | 27.98 | 27.69 | -1.544 | 30.460 | 1.791 | **2** | | |
| 17 | 28.69 | - | 30.44 | 29.32 | 28.66 | 28.20 | 27.84 | 27.54 | -1.616 | 30.440 | 2.953 | **3** | | |
| 18 | 28.47 | - | 30.61 | 29.52 | 28.88 | 28.43 | 28.08 | 27.79 | -1.573 | 30.610 | 3.899 | **4** | | |
| 19 | 28.11 | - | 30.35 | 29.39 | 28.83 | 28.43 | 28.12 | 27.87 | -1.385 | 30.350 | 5.038 | **5** | | |
| 20 | 27.97 | - | 30.44 | 29.49 | 28.93 | 28.54 | 28.23 | 27.98 | -1.371 | 30.440 | 6.061 | **6** | | |

### Example 13: SNP and CNV Analysis Using Human Genomic DNA

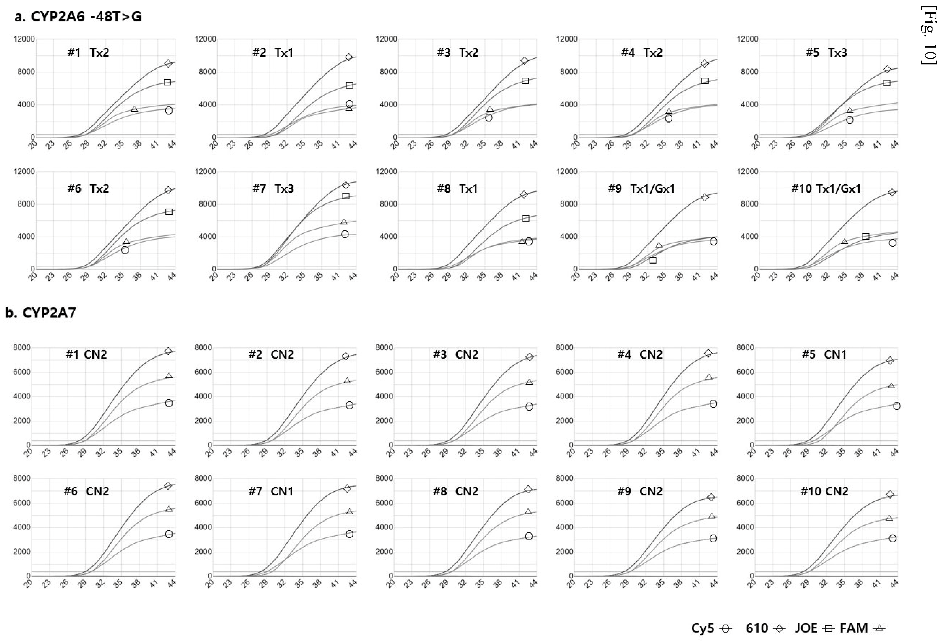

In the above various embodiments, it was confirmed that SNP typing and copy number measurement of CYP2A6 were accurately performed using the standard plasmids. The test method thus constructed was tested using human genomic DNA as clinical samples. The human genomic DNA was quantified, and approximately 5 ng was added to each reaction well for testing. At this time, the primers/probes and qPCR were performed in the same manner as in Example 5 and Example 10. The test was performed using 100 human genomic DNA samples, and representative test results for 10 samples are presented (FIG. 10, FIG. 11, FIG. 12, and Table 13). As a result of this test, diplotypes were clearly identified according to the clinical samples, and the analysis results showed that the results of each of the three replicate tests were mutually similar and were determined to have the same CN and diplotype, as shown in FIG. 11b and Table 13c. The analysis results using the averaged values of the replicate test results were also determined to have the same diplotype as the results of the individual tests (FIG. 11c and Table 14). Through accurate determination of the copy number of the target gene CYP2A6, determination of allele type (SNP, etc.), and determination of the presence or absence of the pseudogene CYP2A7 and its copy number, the diplotype of the target gene CYP2A6 could be clearly determined. For this determination, the CN factor is determined through the methods of Examples 7, 9, and 10, and, based thereon, the CN Ct values are calculated and obtained, and using the same, a calibration curve is obtained to derive a natural logarithmic function y = A × ln(x) + B, which can be calculated respectively as in the example of sample 1 (FIGS. 13a and 13b). Using the first test result of sample 1 (Table 13) as an example, the dCt (1.12 = 28.57 - 27.45) of CN2 (IC1, Cy5) and CN4 (IC2, CFR610) was obtained, and using the same, the theoretical Ct factors of Example 9 (Table 7) and Example 11 (Table 10) were applied to obtain the respective CN Ct values (CN1, 29.69; CN2, 28.57; CN3, 27.91; CN4, 27.45; CN5, 27.09; CN6, 26.79). Using the Ct values of each CN, a calibration curve was prepared as shown in FIG. 13, and a regression analysis equation thereof, y = -1.616 × ln(x) + 29.69, was obtained. From this equation, by substituting Ct(FAM) or Ct(JOE) for y, x, i.e., the copy number, can be easily obtained. That is, the calculation of the total copy number of CYP2A6 in sample 1 may be performed as follows:

From the equation 28.62 (Ct_{[FAM]}) = -1.616 × ln (copy number of #1) + 29.69(Ct_{[CN1]}), ln (CN of #1) = (29.69 - 28.62)/1.616 = 0.662, CN of #1 (CYP2A6) = EXP(0.662), i.e., 1.959 copies (calculated copy number).

Therefore, the total copy number of sample number 1 CYP2A6 gene can be determined to be 2. This was determined identically to the results of the repeat test (Table 13) or the average test (Table 14).

By applying the same method, the [T] allele copy number using Ct_{(JOE)} (Tables 13 and 14) and the total copy number of CYP2A7 using Ct_{(FAM)} of the CYP2A7 assay (Table 14) could also be precisely measured. Using the total CYP2A6 copy number and [T] allele copy number thus obtained, the diploid genotype of CYP2A6 may be accurately determined, and furthermore, the gene number of CYP2A7, which is a pseudogene of CYP2A6 in the sample, may also be clearly determined. As explained by the example of sample No. 1, it can be determined that the total CYP2A6 copy number is 2, the [T] allele copy number is also 2, which is a diploid genotype Tx2, and the total CYP2A7 copy number is also 2.

Table 13 is a calibration curve derivation for copy number calculation and a copy number calculation table calculated therefrom (human genome test - repeat test).

**[Table 13]**

| **CYP2A6 -48T>G** | | | | | | | | | **y = A x ln (x) +B** | | **Total Copy Number** | | **[T] Allele Copy Number** | |
|---|---|---|---|---|---|---|---|---|---|---|---|---|---|---|
| Sampl e No. | **FAM** | **JOE** | CN 1 | **CN 2 (Cy5)** | CN 3 | **CN 4 (610)** | CN 5 | CN 6 | A | B | Calcul ated | **Jud gem ent** | Calcu lated | **Jud gem ent** |
| 1 | **28.62** | **28.52** | **29.69** | **28.57** | **27.91** | **27.45** | **27.09** | **26.79** | -1.616 | 29.690 | 1.939 | **2** | 2.063 | **2** |
| | 28.49 | 28.36 | 29.73 | 28.58 | 27.91 | 27.43 | 27.06 | 26.76 | -1.659 | 29.730 | 2.111 | **2** | 2.283 | **2** |
| | 28.43 | 28.29 | 29.43 | 28.33 | 27.69 | 27.23 | 26.88 | 26.59 | -1.587 | 29.430 | 1.878 | **2** | 2.051 | **2** |
| 2 | 30.18 | 30.01 | 30.14 | 29.11 | 28.51 | 28.08 | 27.75 | 27.48 | -1.486 | 30.140 | 0.974 | **1** | 1.092 | **1** |
| | 30.01 | 29.76 | 30.12 | 29.07 | 28.46 | 28.02 | 27.68 | 27.40 | -1.515 | 30.120 | 1.075 | **1** | 1.268 | **1** |
| | 30.16 | 29.9 | 29.97 | 28.97 | 28.39 | 27.97 | 27.65 | 27.38 | -1.443 | 29.970 | 0.877 | **1** | 1.050 | **1** |
| 3 | 29.45 | 29.28 | 30.45 | 29.35 | 28.71 | 28.25 | 27.90 | 27.61 | -1.587 | 30.450 | 1.878 | **2** | 2.090 | **2** |
| | 29.39 | 29.29 | 30.49 | 29.39 | 28.75 | 28.29 | 27.94 | 27.65 | -1.587 | 30.490 | 2.000 | **2** | 2.130 | **2** |
| | 29.6 | 29.45 | 30.53 | 29.40 | 28.74 | 28.27 | 27.91 | 27.61 | -1.631 | 30.530 | 1.769 | **2** | 1.940 | **2** |
| 4 | 29.44 | 29.31 | 30.31 | 29.20 | 28.55 | 28.09 | 27.73 | 27.44 | -1.602 | 30.310 | 1.722 | **2** | 1.867 | **2** |
| | 29.29 | 29.15 | 30.18 | 29.12 | 28.50 | 28.06 | 27.72 | 27.44 | -1.530 | 30.180 | 1.790 | **2** | 1.961 | **2** |
| | 29.6 | 29.5 | 30.44 | 29.32 | 28.66 | 28.20 | 27.84 | 27.54 | -1.616 | 30.440 | 1.682 | **2** | 1.789 | **2** |
| 5 | 28.88 | 28.72 | 30.42 | 29.28 | 28.61 | 28.14 | 27.77 | 27.47 | -1.645 | 30.420 | 2.550 | **3** | 2.811 | **3** |
| | 28.64 | 28.47 | 30.49 | 29.35 | 28.68 | 28.21 | 27.84 | 27.54 | -1.645 | 30.490 | 3.079 | **3** | 3.415 | **3** |
| | 28.55 | 28.48 | 30.10 | 29.06 | 28.45 | 28.02 | 27.69 | 27.41 | -1.501 | 30.100 | 2.809 | **3** | 2.943 | **3** |
| 6 | 29.16 | 29.08 | 30.25 | 29.19 | 28.57 | 28.13 | 27.79 | 27.51 | -1.530 | 30.250 | 2.040 | **2** | 2.149 | **2** |
| | 28.93 | 28.85 | 29.93 | 28.96 | 28.39 | 27.99 | 27.68 | 27.42 | -1.400 | 29.930 | 2.043 | **2** | 2.163 | **2** |
| | 29.09 | 29.12 | 30.01 | 29.02 | 28.44 | 28.03 | 27.71 | 27.45 | -1.429 | 30.010 | 1.904 | **2** | 1.865 | **2** |
| 7 | 27.6 | 27.53 | 29.14 | 28.11 | 27.51 | 27.08 | 26.75 | 26.48 | -1.486 | 29.140 | 2.819 | **3** | 2.955 | **3** |
| | 27.59 | 27.6 | 29.11 | 28.10 | 27.51 | 27.09 | 26.76 | 26.50 | -1.457 | 29.110 | 2.838 | **3** | 2.818 | **3** |
| | 27.71 | 27.68 | 29.17 | 28.12 | 27.51 | 27.07 | 26.73 | 26.45 | -1.515 | 29.170 | 2.621 | **3** | 2.674 | **3** |
| 8 | 29.83 | 29.64 | 29.98 | 28.94 | 28.33 | 27.90 | 27.57 | 27.29 | -1.501 | 29.980 | 1.105 | **1** | 1.254 | **1** |
| | 29.95 | 29.69 | 30.02 | 28.97 | 28.36 | 27.92 | 27.58 | 27.30 | -1.515 | 30.020 | 1.047 | **1** | 1.243 | **1** |
| | 30.19 | 30.1 | 30.12 | 29.04 | 28.41 | 27.96 | 27.61 | 27.33 | -1.558 | 30.120 | 0.956 | **1** | 1.013 | **1** |
| 9 | 29.03 | 29.76 | 29.90 | 28.77 | 28.11 | 27.64 | 27.28 | 26.98 | -1.631 | 29.900 | 1.705 | **2** | 1.090 | **1** |
| | 28.71 | 29.63 | 29.78 | 28.67 | 28.02 | 27.56 | 27.20 | 26.91 | -1.602 | 29.780 | 1.951 | **2** | 1.098 | **1** |
| | 28.61 | 29.57 | 29.57 | 28.49 | 27.86 | 27.41 | 27.06 | 26.78 | -1.558 | 29.570 | 1.852 | **2** | 1.000 | **1** |
| 10 | 28.28 | 29.17 | 29.32 | 28.25 | 27.62 | 27.18 | 26.84 | 26.55 | -1.544 | 29.320 | 1.961 | **2** | 1.102 | **1** |
| | 28.18 | 29.2 | 29.01 | 28.01 | 27.43 | 27.01 | 26.69 | 26.42 | -1.443 | 29.010 | 1.778 | **2** | 0.877 | **1** |
| | 28.1 | 29.13 | 28.96 | 27.98 | 27.41 | 27.00 | 26.68 | 26.43 | -1.414 | 28.960 | 1.837 | **2** | 0.887 | **1** |

Table 14 is a calibration curve derived for copy number calculation and a copy number calculation table calculated therefrom (human genome test - average value).

**[Table 14]**

| **CYP2A6 (-48T>G)** | | | | | | | | | ***y* = A x ln(x) + B** | | **Total Copy Number** | | **[T] Allele Copy Number** | |
|---|---|---|---|---|---|---|---|---|---|---|---|---|---|---|
| Sampl e No. | **FAM** | **JOE** | CN1 | CN2 (Cy5) | CN3 | CN4 (610) | CN5 | CN6 | A | B | Calcul ated | **Jud gem ent** | Calcu lated | **Jud gem ent** |
| 1 | 28.51 | 28.39 | 29.61 | 28.49 | 27.83 | 27.37 | 27.01 | 26.71 | -1.616 | 29.610 | 1.975 | **2** | 2.128 | **2** |
| 2 | 30.12 | 29.89 | 30.08 | 29.05 | 28.45 | 28.02 | 27.69 | 27.42 | -1.486 | 30.080 | 0.974 | **1** | 1.137 | **1** |
| 3 | 29.48 | 29.34 | 30.49 | 29.38 | 28.73 | 28.27 | 27.91 | 27.62 | -1.602 | 30.490 | 1.879 | **2** | 2.050 | **2** |
| 4 | 29.44 | 29.32 | 30.30 | 29.21 | 28.57 | 28.12 | 27.77 | 27.48 | -1.573 | 30.300 | 1.728 | **2** | 1.865 | **2** |
| 5 | 28.69 | 28.56 | 30.34 | 29.23 | 28.58 | 28.12 | 27.76 | 27.47 | -1.602 | 30.340 | 2.802 | **3** | 3.039 | **3** |
| 6 | 29.06 | 29.02 | 30.07 | 29.06 | 28.47 | 28.05 | 27.72 | 27.46 | -1.457 | 30.070 | 2.000 | **2** | 2.056 | **2** |
| 7 | 27.63 | 27.6 | 29.14 | 28.11 | 27.51 | 27.08 | 26.75 | 26.48 | -1.486 | 29.140 | 2.762 | **3** | 2.819 | **3** |
| 8 | 29.99 | 29.81 | 30.03 | 28.98 | 28.37 | 27.93 | 27.59 | 27.31 | -1.515 | 30.030 | 1.027 | **1** | 1.156 | **1** |
| 9 | 28.78 | 29.65 | 29.74 | 28.64 | 28.00 | 27.54 | 27.19 | 26.90 | -1.587 | 29.740 | 1.831 | **2** | 1.058 | **1** |
| 10 | 28.19 | 29.17 | 29.10 | 28.08 | 27.48 | 27.06 | 26.73 | 26.46 | -1.472 | 29.100 | 1.856 | **2** | 0.954 | **1** |

| CYP2A7 | | | | | | | | | *y* = A x ln(x) +B | | Total Copy Number | | - | |
|---|---|---|---|---|---|---|---|---|---|---|---|---|---|---|
| Sampl | **FAM** | **JOE** | CN1 | CN2 | CN3 | CN4 | CN5 | CN6 | A | B | Calcul | **Jud** | | |
| e No. | | | | (Cy5) | | (610) | | | | | ated | **gem ent** | | |
| 1 | 28.89 | - | 29.87 | 28.89 | 28.32 | 27.91 | 27.59 | 27.34 | -1.414 | 29.870 | 2.014 | **2** | | |
| 2 | 29.74 | - | 30.74 | 29.74 | 29.16 | 28.74 | 28.42 | 28.15 | -1.443 | 30.740 | 2.099 | **2** | | |
| 3 | 30.04 | - | 30.99 | 30.00 | 29.42 | 29.01 | 28.69 | 28.43 | -1.429 | 30.990 | 1.945 | **2** | | |
| 4 | 29.78 | - | 30.78 | 29.78 | 29.20 | 28.78 | 28.46 | 28.19 | -1.443 | 30.780 | 1.959 | **2** | | |
| 5 | 29.76 | - | 30.76 | 29.76 | 29.18 | 28.76 | 28.44 | 28.17 | -1.443 | 30.760 | 1.035 | **1** | | |
| 6 | 29.94 | - | 30.92 | 29.94 | 29.37 | 28.96 | 28.64 | 28.39 | -1.414 | 30.920 | 2.101 | **2** | | |
| 7 | 28.99 | - | 30.01 | 28.99 | 28.39 | 27.97 | 27.64 | 27.37 | -1.472 | 30.010 | 1.123 | **1** | | |
| 8 | 29.59 | - | 30.64 | 29.59 | 28.98 | 28.54 | 28.20 | 27.92 | -1.515 | 30.640 | 2.067 | **2** | | |
| 9 | 29.25 | - | 30.-26 | 29.25 | 28.66 | 28.24 | 27.91 | 27.65 | -1.457 | 30.260 | 2.028 | **2** | | |
| 10 | 28.98 | - | 29.98 | 28.98 | 28.40 | 27.98 | 27.66 | 27.39 | -1.443 | 29.980 | 2.028 | **2** | | |

### Example 14: Analysis results of CYP2A6(-48T>G) and CYP2A7 in 100 Korean samples

To confirm the usefulness of the genotyping and copy number determination method of the present invention, 100 Korean clinical genomic DNA samples obtained from the Daejeon Biomedical Regulatory-Free Zone Human-Derived Material Bank were tested as in Example 13, and the results shown in Table 15 were obtained. As a result of the test, copy number variations of CYP2A6 were observed in a total of 32 samples (32%), among which copy number decreases were observed in 19 individuals (19%) and copy number increases were observed in 13 individuals (13%). The total number of alleles calculated based thereon was 194 (111 + 8 + 40 + 35 = 194), the ratio of allele [T] was 0.778 (151/194), and the ratio of allele [G] was 0.221 (43/194). In particular, since CYP2A7 exhibited only the T allele (T = 186), when this was reflected, the T frequency was calculated as 0.887 ((151 + 186)/380) and the allele [G] frequency was calculated as 0.113 (43/380). These frequencies were very similar to the Asian (T = 0.863, G = 0.137) and East Asian (T = 0.864, G = 0.136) frequencies reported in the NIH dbSNP database, and it was determined that previously reported results showed slight differences from the results of the present example because the copy number of CYP2A7 could not be excluded (Table 16).

In addition, analysis of CYP2A7 in genomic DNA confirmed that the copy number of said pseudogene was highly correlated with the CYP2A6 (-48T>G) diplotypes (Table 17). Among samples in which the total copy number of CYP2A6 was decreased or increased, a total of 16 subjects exhibited variations in the total copy number of CYP2A6, of which 7 of the 8 Tx3 Type subjects (excluding #93) (88%) and all three Tx2/Gx1 Type subjects (100%) were determined to have a variation with one copy of CYP2A7 (Table 17). This analysis result clearly reflects crossover between CYP2A6 and CYP2A7 having similar sequences, and demonstrates that the utility of the analysis method of the present invention is very high.

Table 15 shows copy number frequencies according to CYP2A6-48T>G variants in Koreans.

**[Table 15]**

| | | | | | | |
|---|---|---|---|---|---|---|
| **T homo** | Diplotype (Total Copy Number) | Tx1 homo (1) | Tx2 homo (2) | Tx3homo (3) | Tx4 homo (4) | **58** (T:111) |
| | Korean, n | 15 | 34 | 8 | 1 | |
| **G homo** | Diplotype (Total Copy Number) | Gx1 homo (1) | Gx2 homo (2) | - | | **6** (G:8) |
| | Korean, n | 4 | 2 | | | |
| **Hetero** | Diplotype (Total Copy Number) | Tx1/Gx1 (2) | Tx2/Gx1 (3) | Tx3/ Gx1 (4) | ND | **36** (T:40, G:35) |
| | Korean, n | 31 | 3 | 1 | 1 | |
| **Total** | | **100** (T:151, G:43) | | | | |

Table 16 compares the copy number frequencies by CYP2A6(-48T>G) variant (Korean).

**[Table 16]**

| Population | Sample Size | Ref Allele frequency | Alt Allele frequency | Data from |
|---|---|---|---|---|
| Korean | 100 | T = 0.778 | G = 0.221 | This Work |
| Asian | 204 | T = 0.863 | G = 0.137 | NIH dbSNP* |
| East Asian | 154 | T = 0.864 | G = 0.136 | |
| Total | 40856 | T = 0.94716 | G = 0.05284 | |

| | | | | |
|---|---|---|---|---|
| *https:/www.ncbi.nlm.nih.gov/snp/rs28399433; release version 20201027095038 | | | | |

Table 17 shows the correlation between CYP2A7 copy number variations and CYP2A6 copy numbers.

**[Table 17]**

| Sample No. | 5 | 7 | 16 | 27 | 33 | 34 | 36 | 46 | 66 | 67 | 71 | 87 | 96 | 102 | 108 | 113 |
|---|---|---|---|---|---|---|---|---|---|---|---|---|---|---|---|---|
| CYP2A6-48T>G Diplotype | Tx3 | Tx3 | Tx3 | Tx2 | Tx3 | Tx2 Gx1 | Tx2 | Tx3 | Tx2 Gx1 | Tx1 | Tx3 | Tx2 | Tx3 | Tx2 Gx1 | Tx2 | Tx1 |
| CYP2A 7 Copy Number | 1 | 1 | 1 | 1 | 1 | 1 | 3 | 1 | 1 | 1 | 1 | 1 | 1 | 1 | 1 | 1 |
| - Population: Korean | | | | | | | | | | | | | | | | |
| - Sample Size: 100 | | | | | | | | | | | | | | | | |
| - CYP2A7 Variants: 16 | | | | | | | | | | | | | | | | |

### Industrial Applicability

The method and kit for simultaneous determination of gene copy number and gene variant of the present invention apply real-time polymerase chain reaction to simultaneously determine a gene variant or allele type from a trace amount of a biological sample and to determine the copy number of a target gene, and thus can be widely used in genetic testing of agricultural products, aquatic products, and livestock products, as well as in diagnoses in the medical field such as cancer and genetic diseases.

### Sequence Listing Free Text

An electronic file is attached.

## Claims

1. A method for simultaneously determining a copy number and a genotype of a gene, comprising:
a) obtaining amplification experimental values (Ct values) by qPCR by simultaneously adding to one reaction tube: i) a target gene-specific primer, a target gene-specific probe, and a probe capable of distinguishing alleles of the target gene; and ii) a reference gene-specific primer and probe capable of amplifying and detecting a reference gene, and synchronizing amplification of the genes;
b) determining a copy number Ct factor (CN Ct factor) from the amplification experimental values and/or theoretical amplification values;
c) determining Ct values of target gene copy numbers 1, 3, 5 and/or 6 from amplification experimental values of copy number 2 and copy number 4 of the reference gene by using the determined copy number Ct factor; and
d) determining the total copy number of the target gene and the genotype of alleles by comparing amplification experimental values of the target gene with the Ct values of copy numbers 1, 3, 5 and/or 6 of the target gene determined in step c) and the test copy number of the reference gene.

2. The method of claim 1, wherein
in step a), in i), a primer and a probe capable of specifically amplifying a pseudogene of the target gene are further added.

3. The method of claim 1 or 2, wherein
at least one of the target gene-specific primer and the pseudogene-specific primer is an AS primer (Allele specific primer) or an ARMS primer (Amplification Refractory Mutation System primer).

4. The method of any one of claims 1 to 3, wherein
in the qPCR step of step a),
a discrimination-enhancing oligonucleotide that is not complementary to the primers and the template, is capable of reversibly binding to a DNA polymerase for qPCR, and is configured to form a partially or fully double-stranded structure within a single strand or with another strand, is additionally introduced to enhance discrimination of specific primer amplification; and
a non-specific signal-suppressing probe that is complementary to a target nucleotide sequence of a non-variant target gene, preferentially binds to the target nucleotide sequence of the non-variant target gene over a target nucleotide sequence of a variant target gene, and has a structure whose 3' end is not extended by a nucleic acid polymerase, is additionally introduced to enhance discrimination of a specific probe.

5. The method of claim 1, wherein
in the qPCR step of step a), for confirming a copy number of the target gene and an allele copy number, qPCR is performed using a target gene-specific probe and an allele-specific probe that are fluorescent probes having different wavelengths.

6. The method of claim 1, wherein
in the qPCR step of step a), for obtaining amplification experimental values of copy number 2 and copy number 4 of the reference gene, qPCR is performed using fluorescent probes having different wavelengths.

7. The method of claim 1, wherein
in step b), the determining of the copy number Ct factor from the amplification experimental values is performed by deriving a calibration curve using experimental Ct values of a standard gene and determining copy number Ct factors for copy numbers 1, 3, 5 and/or 6 of the target gene.

8. The method of claim 1, wherein
in step b), the determining of the copy number Ct factor from the theoretical amplification values is performed using Equation y = -1.443 × ln(x) + 1, wherein y is a Ct value of the target gene and x is a copy number of the target gene.

9. The method of claim 1, wherein
in step c), the determining of Ct values of target gene copy numbers using the copy number Ct factor is performed by calculating a difference (dCt(CN2-CN4)) between a Ct of copy number 2 and a Ct of copy number 4 of the reference gene obtained from amplification experimental values of the target gene and the reference gene having known copy numbers, and applying the copy number Ct factor obtained in claim 6 or 7 to determine Ct for copy numbers 1, 3, 5 and/or 6 of the target gene.

10. The method of claim 9, wherein
the comparing of amplification experimental values of the target gene with Ct values of target gene copy numbers is performed by deriving a standard quantitative curve equation, y = A × ln(x) + B, by applying Ct values of copy numbers 1, 3, 5 and/or 6 of the target gene and a CN2 Ct value and a CN4 Ct value of the target gene, wherein y is a Ct value of the target gene and x is a copy number of the target gene, and determining copy numbers of the target gene and alleles using the equation.

11. The method of claim 1, wherein
the synchronizing amplification of the target gene and the reference gene is performed by conducting qPCR with the target gene and the reference gene located on a single plasmid.

12. The method of claim 1, wherein
the performing qPCR by synchronizing amplification of the target gene and the reference gene is carried out by adjusting qPCR conditions so that Ct value deviations dCt_{(TCN-IC1)} and dCt_{(AS-IC1)} each satisfy ±0.1 at the same gene copy number for both genes in the qPCR reaction of the reference gene and the target gene.

13. The method of claim 1, wherein
qPCR is performed such that the copy number of the reference gene is twice the copy number of the target gene.

14. The method of claim 13, wherein
qPCR is performed by adjusting qPCR conditions so that dCt_{(CN2;IC1-CN4;IC2)} satisfies 1.0 ± 0.1 when the copy number of the reference gene is twice the copy number of the target gene.

15. The method of claim 6 or 14, wherein
in the qPCR step, for obtaining amplification experimental values of copy number 4, two probes having different sequences and the same fluorescence are simultaneously used.

16. The method of claim 1, wherein
for obtaining amplification experimental values of copy number 4 of the target gene, a Ct value that is 1.0 less than a Ct of copy number 2 of the target gene is arbitrarily substituted and calculated as a Ct of copy number 4.

17. A method for preparing a simultaneous determination table of a gene copy number and a genotype, comprising:
displaying qPCR Ct results and copy number Ct values on one axis;
displaying target gene samples to be analyzed on another axis; and
displaying the table such that a genotype and a copy number of one or more analysis samples are visually identifiable.

18. The method of claim 17, wherein
the determination table comprises Ct values, Ct values by copy number, and copy numbers of diplotypes and allele types.

19. The method of claim 18, wherein
the determination table further comprises a copy number of a pseudogene.

20. The method of any one of claims 17 to 19, wherein
the determination table reflects result values and/or calculated values obtained from the method of any one of claims 1 to 16.

21. A simultaneous determination table of a gene copy number and a genotype,
displaying qPCR Ct results and copy number Ct values on one axis and displaying target gene samples to be analyzed on another axis such that a genotype and a copy number of one or more analysis samples are visually identifiable.

22. The simultaneous determination table of claim 21, wherein
the determination table comprises Ct values, Ct values by copy number, and copy numbers of diplotypes and allele types.

23. The simultaneous determination table of claim 22, wherein
the determination table further comprises a copy number of a pseudogene.

24. A qPCR kit for simultaneously determining a copy number and a genotype of a
target gene, comprising:
a) a target gene-specific primer and a target gene-specific probe;
b) a specific probe capable of distinguishing alleles of the target gene;
c) a reference gene-specific primer and a reference gene-specific probe capable of amplifying and detecting a reference gene; and
d) a DNA polymerase.

25. The qPCR kit of claim 24, further comprising:
e) a primer and a probe capable of specifically amplifying a pseudogene of the target gene.

26. The qPCR kit of claim 24 or 25, further comprising:
f) a discrimination-enhancing oligonucleotide that is not complementary to the primers and a template of the target gene, is capable of reversibly binding to a DNA polymerase for qPCR, and is configured to form a partially or fully double-stranded structure within a single strand or with another strand; and
g) a non-specific signal-suppressing probe that is complementary to a target nucleotide sequence of a non-variant target gene, preferentially binds to the target nucleotide sequence of the non-variant target gene over a target nucleotide sequence of a variant target gene, and has a structure whose 3' end is not extended by a nucleic acid polymerase.
